(19) Europäisches Patentamt
European Patent Office
Office européen des brevets

(11) **EP 4 368 195 A1**

(12) **EUROPEAN PATENT APPLICATION**

(43) Date of publication:
**15.05.2024  Bulletin 2024/20**

(21) Application number: **22207154.0**

(22) Date of filing: **14.11.2022**

(51) International Patent Classification (IPC):
**A61K 38/14** (2006.01)    **A61K 31/505** (2006.01)
**A61K 31/519** (2006.01)    **A61K 31/7036** (2006.01)
**A61K 45/06** (2006.01)    **A61P 31/04** (2006.01)

(52) Cooperative Patent Classification (CPC):
(C-Sets available)
**A61K 45/06; A61K 31/505; A61K 31/519;
A61K 31/7036; A61K 38/14; A61P 31/04;**
A61K 2300/00                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **European Molecular Biology
Laboratory
69117 Heidelberg (DE)**

(72) Inventors:
• **TYPAS, Athanasios
69120 Heidelberg (DE)**

• **CACACE, Elisabetta
53100 Siena (IT)**
• **GÖTTIG, Stephan
63263 Neu-Isenburg (DE)**

(74) Representative: **Krauss, Jan
SKM-IP PartGmbB
Oberanger 45
80331 München (DE)**

Remarks:
The complete document including Reference
Table(s) and the Sequence Listing(s) can be
downloaded from the EPO website

(54) **NOVEL COMBINATIONS OF ANTIBIOTICS AND NON-ANTIBIOTIC DRUGS EFFECTIVE IN VIVO AGAINST GRAM-POSITIVE BACTERIA, IN PARTICULAR METHICILLIN-RESISTANT S. AUREUS (MRSA)**

(57)    The present invention relates to the field of therapeutics and in particular to pharmaceutical compositions for the prevention and/or treatment of bacterial infections. The compositions of the present invention demonstrate a synergistic effect in inhibiting the growth of the most clinically relevant Gram-positive bacterial species, and resistant clinical isolates thereof (e.g. MRSA). The two combinations illustrate two novel principles in antimicrobial treatments, using non-antibiotic drugs as adjuvants and decoupling known fixed-dose combinations to repurpose their individual members in novel combinations. The combinations demonstrated a strong synergistic inhibitory effect on the growth, the killing activity and the *in vivo* infection outcome of clinically relevant Gram-positive pathogens, such as MRSA. Importantly, this invention relates to combinations that target multi-drug resistant strains, constituting a major effort of antimicrobial therapy development in the current antimicrobial resistance crisis.

**EP 4 368 195 A1**

(52) Cooperative Patent Classification (CPC): (Cont.)

C-Sets
A61K 2300/00, A61K 31/505, A61K 38/14;
A61K 2300/00, A61K 31/519, A61K 31/7036

**Description**

The present invention relates to the field of therapeutics and in particular to pharmaceutical compositions for the prevention and/or treatment of bacterial infections. The compositions of the present invention demonstrate a synergistic effect in inhibiting the growth of the most clinically relevant Gram-positive bacterial species, and resistant clinical isolates thereof (e.g. MRSA). The two combinations illustrate two novel principles in antimicrobial treatments, using non-antibiotic drugs as adjuvants and decoupling known fixed-dose combinations to repurpose their individual members in novel combinations. The combinations demonstrated a strong synergistic inhibitory effect on the growth, the killing activity and the *in vivo* infection outcome of clinically relevant Gram-positive pathogens, such as MRSA. Importantly, this invention relates to combinations that target multi-drug resistant strains, constituting a major effort of antimicrobial therapy development in the current antimicrobial resistance crisis.

**Background of the invention**

Antibacterial agents have been used in combination since the dawn of the antibiotic era for different purposes: to achieve synergy (e.g. sulfamethoxazole-trimethoprim), to limit resistance (e.g. combinations of beta-lactams and beta-lactamase inhibitors, or antitubercular regimens), and/or to broaden the spectrum of action of anti-infective treatments (e.g. empiric treatments of sepsis). With antimicrobial resistance (AMR) posing a global threat to public health which permeates all domains of modern medicine, the use of drug combinations to re-sensitize resistant strains has emerged as one of the promising means to bypass the stagnant drug discovery pipeline (Tyers, M. and Wright, G. D. Drug combinations: a strategy to extend the life of antibiotics in the 21st century. Nat. Rev. Microbiol. 17, 141-155 (2019)).

Although a few antibacterial combinations are used in clinics in fixed-dosages, and screens for approved compounds as adjuvants for antibiotics have been increasingly conducted in the last decade, the full potential of drug combinations for treating bacterial pathogens remains underexplored. This is because the combinatorial space is vast, and drug interactions are rare and concentration-, drug-, time-, species- and even strain-specific, making systematic testing necessary, yet highly demanding. As a result, drug interactions have not yet been systematically profiled in many clinically relevant bacterial species.

With the increase of polypharmacy, antibiotics are prescribed in combination with other drugs in 61.7% of the cases (Centers for Disease Control and Prevention (CDC). National Center for Health Statistics (NCHS). National Health and Nutrition Examination Survey Data. Hyattsville, MD: U.S. Department of Health and Human Services, Centers for Disease Control and Prevention, 2021, https://wwwn.cdc.gov/nchs/nhanes/2017-2018/p_rxq_rx.htm). While host-driven pharmacokinetic interactions between antibiotics and non-antibiotic drugs (e.g. depending on drug metabolism and excretion by the liver and the kidney) are well-characterized, drug interactions based on bacterial determinants remain poorly understood.

Traub WH, et al. (in: Teicoplanin Combined with Various Antibiotics and Human Blood against a Multiple-Drug-Resistant Strain of Staphylococcus aureus. Chemotherapy. 1991;37(3):186-95. doi: 10.1159/000238852. PMID: 1832374) use *in vitro* killing assays to investigate additive or synergistic effects of combinations of teicoplanin with cotrimoxazole (sulfamethoxazole-trimethoprim) in blood or Mueller-Hinton broth (MHB), revealing mild antagonism in blood at 4 hours and potentiation at 22 hours in two MDR *S. aureus* isolates. No drug-drug interaction could be quantified in MHB because teicoplanin alone achieved complete killing at 22h. This begs the question on (i) whether synergy can be achieved unlocking a novel combinatorial space by decoupling fixed-drug/dose combinations, i.e. decoupling trimethoprim-sulfa combinations, and combining trimethoprim directly with teicoplanin; (ii) whether synergy occurs more generally across several, recently-emerged MRSA clinical isolates; (iii) whether this synergy holds *in vivo*.

WO 2019/206781 relates to the field of therapeutics and, more in particular, to pharmaceutical compositions for the prevention and/or treatment of bacterial infections and antibacterial-induced dysfunctions. The compositions as described demonstrate high species-specificity in inhibiting the growth of a small number of bacterial species, and most importantly are effective also against multi drug resistant (MDR) clinical isolate species. Interestingly, one of those combinations pairs a non- antibiotic drug, vanillin, with an antibiotic drug, spectinomycin, to demonstrate a surprisingly strong inhibitory effect on the growth of clinically relevant Gram-negative pathogenic and multi-drug resistant E. coli isolates. A second set of compounds combines the polymyxin colistin with loperamide, a rifamycin, or a macrolide.

Wu W, et al. (in: Teicoplanin combined with conventional vancomycin therapy for the treatment of pulmonary methicillin-resistant Staphylococcus aureus and Staphylococcus epidermidis infections. World J Clin Cases. 2021 Dec 6;9(34):10549-10556. doi: 10.12998/wjcc.v9.i34.10549. PMID: 35004986; PMCID: PMC8686121) explore the value of teicoplanin combined with conventional (vancomycin only) anti-infective therapy for the treatment of methicillin-resistant *Staphylococcus aureus* and *Staphylococcus epidermidis* pulmonary infections. They conclude that compared with conventional (vancomycin only) therapy, teicoplanin and vancomycin combination therapy for patients with pulmonary methicillin-resistant *Staphylococcus aureus* and methicillin-resistant *Staphylococcus epidermidis* infections can improve patient clinical symptoms, modulate serum inflammatory factor levels, and improve treatment efficacy, without increasing the risk of adverse reactions.

Tsuji and Rybak (in: Short-course gentamicin in combination with daptomycin or vancomycin against Staphylococcus aureus in an in vitro pharmacodynamic model with simulated endocardial vegetations. Antimicrob Agents Chemother. 2005 Jul;49(7):2735-45. doi: 10.1128/AAC.49.7.2735-2745.2005. PMID: 15980344; PMCID: PMC1168654) analyze short-course regimens of gentamicin in combination with daptomycin or vancomycin against one methicillin-susceptible (MSSA 1199) and one methicillin-resistant (MRSA 494) *Staphylococcus aureus* isolate using an in vitro pharmacodynamic model with simulated endocardial vegetations over 96 h. They suggest that a single high dose of gentamicin in combination with daptomycin or vancomycin may be of utility to maximize synergistic and bactericidal activity and minimize toxicity, nevertheless that further investigation is warranted.

Lancellotti P, et al. (in: Antibacterial Activity of Ticagrelor in Conventional Antiplatelet Dosages Against Antibiotic-Resistant Gram-Positive Bacteria. JAMA Cardiol. 2019 Jun 1;4(6):596-599. doi: 10.1001/jamacardio.2019.1189. PMID: 31066863; PMCID: PMC6506905) use in vitro assays and a mouse model to investigate antibacterial properties of ticagrelor to follow up findings from two clinical trials that showed patients taking ticagrelor for cardiovascular disease had a lower risk of infection-related death and improved lung function compared with patients treated with clopidogrel. A subminimal bactericidal concentration of ticagrelor (10 $\mu$g/mL) combined with vancomycin (4 $\mu$g/mL) killed approximately 50% of the initial MRSA inoculum, depicting synergistic activity. This begs the question whether (i) ticagrelor synergizes with other antibiotics, (ii) whether these synergies hold *in vivo* and (iii) what is their mechanism.

The development and spread of antimicrobial resistance represent one of the most serious threats to public health, with the emergence of bacterial strains that are untreatable with known compounds, or treatable only with drugs bearing strong side-effects on the human host. The Gram-positive bacterial species *S. aureus* and *S. pneumoniae* rank second and fourth, respectively, among the bacterial pathogens with the highest number of deaths associated with antimicrobial resistance worldwide (Global burden of bacterial antimicrobial resistance in 2019: a systematic analysis. Antimicrobial Resistance Collaborators. Lancet. 2022 Feb 12;399(10325):629-655. doi: 10.1016/S0140-6736(21)02724-0). Thus, there is a pressing need to provide novel antibacterial pharmaceutical compositions that can target such resistant strains.

It is therefore an object of the present invention to provide novel antibacterial pharmaceutical compositions. It is a further object of this invention to offer antibacterial pharmaceutical compositions effective against several methicillin-resistant *S. aureus* bacterial strains (MRSA) strains, i.e. clinically relevant Gram-positive pathogens.

This problem is solved by the present invention by providing antibacterial pharmaceutical compositions for the treatment of Gram-positive bacterial infections, comprising a combination of i) at least one drug selected from the group of ADP-receptor-inhibitor antiplatelet agents and antifolate antibiotics used in the clinic only in fixed concentrations and derivatives and pharmaceutically acceptable salts thereof with ii) at least one antibiotic selected from the group of aminoglycosides, cationic peptides, glycopeptides, and derivatives and pharmaceutically acceptable salts thereof.

Specifically, the inventors discovered two novel antimicrobial synergies between antibiotics and non-antibiotic drug classes and their members trimethoprim-teicoplanin and gentamicin-ticagrelor. The synergies are specifically effective against methicillin-sensitive (MSSA) strains and methicillin-resistant *S. aureus* (MRSA) clinical isolates (additionally resistant to linezolid and tigecycline) both *in vitro* and *in vivo.*

In the context of the present invention, the inventors performed a broth microdilution checkerboard assay in high-throughput in order to generate a comprehensive resource of drug interactions in three Gram-positive bacterial species: the pathogens *Staphylococcus aureus* and *Streptococcus pneumoniae,* both included in the WHO priority list of antibiotic-resistant bacteria, and the model organism *Bacillus subtilis.* Compared to previous interaction studies in Gram-positive species, this setup vastly increased the number of drugs, concentrations and strains tested.

Moreover, the inventors probed the interactions of antibiotics with a large panel of non-antibiotic drugs in *S. aureus* to investigate the impact of commonly administered medications on antibiotic efficacy. Thereby, the inventors uncovered both strong synergies that remained effective against MDR *S. aureus* clinical isolates, and widespread antagonisms that could compromise the efficacy of antibiotic treatments.

The inventors of the present invention have found that some of the pharmaceutical pairs identified with the method described in the present invention contain non-antibiotic compounds such as antiplatelet agents, like ticagrelor. such synergistic combinations allow to use lower dosages of antibiotics whose clinical applications are often limited by toxicity, constituting a major effort of current and future drug development in order to prevent major side effects of antibacterial strategies. Other objects of the present invention will become apparent to the person of skill when studying the specification of the present invention.

In a first aspect thereof, the object of the present invention is solved by providing an antibacterial pharmaceutical composition for the treatment of a Gram-positive bacterial infection, comprising a combination of i) at least one drug selected from the group of ADP-receptor-inhibitor antiplatelet agents and antifolate antibiotics used in the clinic only in fixed concentrations and derivatives and pharmaceutically acceptable salts thereof with ii) at least one antibiotic selected from the group of aminoglycosides, cationic peptides, glycopeptides, and derivatives and pharmaceutically acceptable salts thereof.

Preferred is a combination of at least one drug selected from the group of ADP-receptor-inhibitor antiplatelet agents with at least one antibiotic selected from the group of aminoglycosides, cationic peptides and derivatives and pharmaceutically acceptable salts thereof.

Also preferred is a combination of at least one drug from the group of glycopeptides and derivatives and pharmaceutically acceptable salts thereof with at least one drug from the group of antifolate antibiotics used in the clinic only in fixed concentrations and derivatives and pharmaceutically acceptable salts thereof.

As used herein, "synergy" or "synergistic effect" is defined as an antimicrobial effect for a given combination (quantified as growth in broth microdilution assay, number of colony-forming units over time in killing assays, or survival of infected hosts) greater than either drug alone and more than expected according to the neutrality assumption of the Bliss interaction model (Bliss, C. I. THE TOXICITY OF POISONS APPLIED JOINTLY. Ann. Appl. Biol. 26, 585-822 615 (1939)), i.e. the multiplication of the single-drug effects as also described herein. "Synergistic" effects are scored by effect size and adjusted p-values as disclosed below in the "Methods" section of this description.

As used herein, the term "ADP-receptor-inhibitor antiplatelet agent" refers to a compound functioning as inhibitor of thrombocyte aggregation through ADP-receptor-inhibition, such as ticagrelor, and related compounds, and derivatives thereof.

As used herein, the term "adjuvants" refers to a non-antibiotic compound potentiating the action of antibiotics (see, for example, Ejim, L. et al. Combinations of antibiotics and nonantibiotic drugs enhance antimicrobial efficacy. Nat. Chem. Biol. 7, 348-350 (2011)).

As used herein, the term "antibiotics used in the clinic only in fixed concentrations" refers to drugs that are combined with others only in fixed concentrations, such as the dihydropyrimidine antimicrobial trimethoprim.

As used herein, the term "antibiotic selected from the group of glycopeptides" refers to peptides interfering with cell-wall synthesis, such as the anti-infective antibiotics vancomycin, teicoplanin, telavancin, ramoplanin and decaplanin, corbomycin, and complestatin, preferably teicoplanin.

As used herein, the term "antibiotic selected from the group of aminoglycosides" refers to antibiotics interfering with protein synthesis such as gentamicin, tobramycin, amikacin, plazomicin, streptomycin, kanamycin, neomycin, and paromomycin, preferably gentamicin.

As used herein, the term "derivatives" refers to the indicated antibiotic compounds that include modifications by a variety of methods known in the art, which include without limitation the introduction of novel side chains or the exchange of functional groups like introduction of halogens, in particular F, Cl or Br, the introduction of lower alkyl groups, preferably having one to five carbon atoms like methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of $NH_2$, $NO_2$, OH, SH, NH, CN, aryl, heteroaryl, COH or COOH group.

The object of the present invention is solved by providing an antibacterial pharmaceutical composition for the treatment of a Gram-positive bacterial infection, comprising a combination of ticagrelor and gentamicin and derivatives and pharmaceutically acceptable salts thereof.

Preferred are the afore-mentioned methods, wherein said bacterial infection is caused by a Gram-positive bacterium, preferably wherein said Gram-positive bacterial infection is caused by *S. aureus,* and antibiotic-resistant strains thereof, such as MRSA.

A further aspect of the present invention then relates to the pharmaceutical composition according to the present invention for use in the prevention and/or treatment of a bacterial infection, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection.

A further preferred aspect of this invention is a method for producing an antibacterial pharmaceutical composition according to the present invention, comprising the step of formulating said combination as selected into an antibacterial pharmaceutical composition for the treatment of a Gram-positive bacterial infection. Yet another aspect of the present invention relates to an antibacterial pharmaceutical composition, produced according to the afore-mentioned method.

The present invention also relates to a method for preventing and/or treating a bacterial infection in a subject in need thereof, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection, comprising administering to said subject an effective amount of the pharmaceutical composition according to the present invention. Preferably, said bacterial infection is caused by a Gram-positive bacterium, preferably wherein said Gram-positive bacterial infection is by at least one bacterium selected from *S. pneumoniae, B. subtilis, S. aureus,* and antibiotic-resistant strains thereof, such as MRSA.

Preferably, the combination of said pharmaceutical composition is administered to a subject simultaneously, separately, or sequentially, wherein said subject is a mammal, such as a human, preferably a human patient, optionally wherein said composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, nasal agent, inhalation mixture, aerosol, mouthwash, mouth spray, nose spray.

Advantageously, the method according to the present invention and/or the use according to the present invention repurposes drugs that have not been previously used as antimicrobials (e.g. ticagrelor), or antimicrobials used almost exclusively in fixed-dose combinations with other drugs (e.g. trimethoprim), whose combinatorial potential has therefore remained unexplored.

Advantageously, the method according to the present invention and/or the use according to the present invention repurposes drugs whose pharmacokinetic/pharmacodynamic and toxicity profiles are well characterized, therefore minimizing the chances of side-effects in humans.

Advantageously, the method according to the present invention and/or the use according to the present invention is highly effective against a panel of MDR bacterial pathogens, that rank very highly in number of resistance-associated deaths worldwide and for which therapeutic options are currently limited.

Advantageously, the method according to the present invention and/or the use according to the present invention furthermore prevents the development and/or spread of bacterial antibiotic resistance.

Advantageously, the method according to the present invention and/or the use according to the present invention furthermore leverages synergism between compounds to reduce their individual dosages, for drugs whose dose increase is strongly limited in humans by the risk of toxicity.

The afore-mentioned compositions of compounds can generally be used in a wide variety of medical applications, in particular in the prevention and/or treatment of bacterial infections and/or dysbiosis. Preferred is the afore-mentioned composition, wherein said composition is for use in the prevention and/or treatment of a bacterial infection, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection.

Further preferred is the afore-mentioned composition, wherein said bacterial infection to be treated and/or prevented is caused by a Gram-positive bacterium, preferably wherein said Gram-positive bacterial infection is by at least one bacterium selected from *S. pneumoniae, B. subtilis, S. aureus,* and antibiotic-resistant strains thereof, such as MRSA.

Yet another embodiment of the invention pertains to the afore-described composition for use, wherein said components of said composition are administered to a subject simultaneously, separately or sequentially, wherein said subject is a mammal, such as a human, preferably a human patient, optionally wherein said composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray.

In the context of the present invention, the inventors have systematically profiled drug combinations in three prominent Gram-positive bacterial species. The inventors probed multiple compounds belonging to all main antibiotic classes used to treat infections caused by Gram-positive pathogens, as well as neglected antibiotics, commonly used antibiotic adjuvants, and promising non-antibiotic drugs with reported antibacterial activity. In all cases combinations were tested in a dose-dependent manner and interactions were assessed in a quantitative manner. This effort unraveled a plethora of interactions, the majority of which are, to the inventors' knowledge, novel. A number of the synergies that the inventors discovered using lab strains were also effective against MDR clinical isolates and during infections *in vivo.*

Overall, the data generated here can seed future experiments to mechanistically dissect key interactions or to explore their potential for clinical application. For example, some of the synergies and antagonisms identified may guide future broad-spectrum empiric treatments - i.e. when antibiotic regimens are started without knowledge of the responsible pathogen in time-sensitive contexts (e.g. sepsis).

The inventors' effort here largely matched a previous large-scale study from the inventors' group in three Gram-negative species (Brochado, A. R. et al. Species-specific activity of antibacterial drug combinations. Nature 559, 259-263 (2018)), which enabled important comparative insights across the Gram-positive/-negative divide. The confidence and depth level of these comparisons are high, since the two studies have very similar experimental and data analysis design (including the drugs tested). As in Gram-negative species, drug interactions are largely species-specific with synergies tending to be more conserved and driven by antibiotics sharing general cellular targets. Only a small number of interactions is conserved across Gram-positive and -negative species. Differences in the cell surface organization (e.g. the outer membrane posing a permeability barrier for hydrophobic compounds) or in the degree of redundancy in cell-wall building enzymes can explain some of the strong synergies observed specifically in either Gram-positive or Gram-negative species.

The inventors also attempted to leverage the adjuvant potential of approved non-antibiotic drugs by probing a large number of combinations with antibiotics (2728) in a dose-dependent manner in *S. aureus.* Although the inventors showed that the interaction potential is lower for drugs without antibacterial activity, the room for novelty is even higher, with the vast majority of synergies detected being previously unknown. While non-antibiotic drugs have been proposed as anti-infective adjuvants for decades, the in vivo relevance and molecular basis of their antibacterial action is known only for a few examples.

Here, the inventors further studied the antiaggregant ticagrelor, whose repurposing as an anti-infective adjuvant for Gram-positive bacteria has been recently proposed (Sun, J. et al. Repurposed drugs block toxin-driven platelet clearance by the hepatic Ashwell-Morell receptor to clear Staphylococcus aureus bacteremia. Sci. Transl. Med. 13, (2021), Phanchana, M. et al. Repurposing a platelet aggregation inhibitor ticagrelor as an antimicrobial against Clostridioides difficile. Sci. Rep. 10, 6497 (2020)). While the in vivo benefit of ticagrelor for systemic infections has been documented (Storey, R. F. et al. Lower mortality following pulmonary adverse events and sepsis with ticagrelor compared to clopidogrel in the PLATO study. Platelets 25, 517-525 (2014), Lancellotti, P. et al. Antibacterial Activity of Ticagrelor in Conventional Antiplatelet Dosages Against Antibiotic-Resistant Gram-Positive Bacteria. JAMA Cardiol 4, 596-599 (2019)), the inventors identified here a large number of additional synergies with antibiotics in *S. aureus* (n = 13), and also provide molecular insights into how ticagrelor affects *S. aureus* physiology and potentiates the activity of positively-charged antibiotics, such as aminoglycosides or nisin.

Drugs are regularly combined in clinics not only in rationally designed therapeutic schemes, but also in an almost serendipitous fashion in poly-treated patients. Although interactions at the host pharmacokinetic level are routinely avoided, it is assumed that interactions at the bacterial level would not contribute to the overall anti-infective efficacy. In addition to synergies, the inventors detected an equally large number of antagonisms between commonly administered non-antibiotic drugs and antibiotics. Such antagonisms could decrease the efficacy of the antibiotic treatment and increase the probability of resistance emergence for the antibiotic. Thus, it is important to start assessing drug interactions at the level of antimicrobial activity. It has been recently proposed that this attenuation of antibiotic efficacy (antagonism) can also be used to decrease the spectrum and the collateral damage of antibiotics (Maier, L. et al. Unravelling the collateral damage of antibiotics on gut bacteria. Nature (2021) doi:10.1038/s41586-021-03986-2). In the inventors' screen, loperamide had the highest number of interactions with antibiotics. Although its potential use as adjuvant for specific antibiotics and its mode-of-action have been previously explored (Ejim, L. et al. Combinations of antibiotics and nonantibiotic drugs enhance antimicrobial efficacy. Nat. Chem. Biol. 7, 348-350 (2011)), the inventors could detect a broad antagonism with macrolides. Loperamide and macrolides are often co-administered for travellers' diarrhea, which is caused by Gram-negative enteric pathogens. It is tempting to speculate that part of the beneficial effect of the combination could also lie in the protection of Gram-positive commensal gut species from macrolides.

The inventors discovered two combinations, never proposed before, that synergize against Methicillin-resistant *Staphylococcus aureus* (MRSA), the most relevant Gram-positive bacterial pathogen in terms of resistance-associated and attributed deaths (Murray, C. J. L. et al. Global burden of bacterial antimicrobial resistance in 2019: a systematic analysis. Lancet 399, 629-655 (2022)). The inventors show that these synergies hold true *in vivo,* protecting the model animal *Galleria mellonella* from infection by MRSA clinical isolates that are additionally resistant to last-resort antibiotics for MRSA, such as tigecycline and linezolid.

The combination of the antibiotics teicoplanin and trimethoprim illustrates how decomposing known combinations unlocks new antimicrobial synergies: trimethoprim is only combined in clinics with sulfonamides, not with the glycopeptide teicoplanin, and is rarely used alone.

The combination of the antiplatelet ticagrelor with the aminoglycoside antibiotic gentamicin epitomizes the potential of non-antibiotic drugs to resensitize resistant strains to antibiotics. While the beneficial effect of ticagrelor during *S. aureus* sepsis has been observed *in vivo* (Lancellotti, P. et al. Antibacterial Activity of Ticagrelor in Conventional Antiplatelet Dosages Against Antibiotic-Resistant Gram-Positive Bacteria. JAMA Cardiol 4, 596-599 (2019)), the potential of its combination with antibiotics has not been explored yet. This is particularly promising for aminoglycosides, whose clinical use is often limited by dose-dependent toxicity. Since the synergistic effect of ticagrelor allows to reduce gentamicin dosage while maintaining and enhancing the overall antimicrobial effect, this combination could represent a safe way to extend and improve aminoglycoside clinical use against MRSA.

The present invention relates to the following items:

Item 1: An antibacterial pharmaceutical composition for the treatment of a Gram-positive bacterial infection, comprising a combination of i) at least one drug selected from the group of ADP-receptor-inhibitor antiplatelet agents and antifolate antibiotics used in the clinic only in fixed concentrations and derivatives and pharmaceutically acceptable salts thereof with ii) at least one antibiotic selected from the group of aminoglycosides, cationic peptides, glycopeptides, and derivatives and pharmaceutically acceptable salts thereof.

Preferred is a combination of at least one drug selected from the group of ADP-receptor-inhibitor antiplatelet agents with at least one antibiotic selected from the group of aminoglycosides, cationic peptides and derivatives and pharmaceutically acceptable salts thereof.

Also preferred is a combination of at least one drug from the group of glycopeptides and derivatives and pharmaceutically acceptable salts thereof with at least one drug from the group of antifolate antibiotics used in the clinic only in fixed concentrations and derivatives and pharmaceutically acceptable salts thereof.

Item 2: The antibacterial pharmaceutical composition for the treatment of a Gram-positive bacterial infection according to Item 1, wherein the ADP-receptor-inhibitors antiplatelet agent is selected from ticagrelor and derivatives and pharmaceutically acceptable salts thereof.

Item 3: The antibacterial pharmaceutical composition for the treatment of a Gram-positive bacterial infection according to Item 1 or 2, wherein the antibiotic used in the clinic only in fixed concentration is selected from trimethoprim, and derivatives and pharmaceutically acceptable salts thereof.

Item 4: The antibacterial pharmaceutical composition for the treatment of a Gram-positive bacterial infection according to any one of Items 1 to 3, wherein the glycopeptide is selected from teicoplanin and derivatives and pharmaceutically acceptable salts thereof.

Item 5: The antibacterial pharmaceutical composition for the treatment of a Gram-positive bacterial infection according to any one of Items 1 to 4, wherein the aminoglycoside is selected from gentamicin and derivatives and pharmaceutically acceptable salts thereof.

Item 6: An antibacterial pharmaceutical composition for the treatment of a Gram-positive bacterial infection, comprising a combination of teicoplanin and trimethoprim and derivatives and pharmaceutically acceptable salts thereof.

Item 7: An antibacterial pharmaceutical composition for the treatment of a Gram-positive bacterial infection, comprising a combination of ticagrelor and gentamicin and derivatives and pharmaceutically acceptable salts thereof.

Item 8: The antibacterial pharmaceutical composition for the treatment of a Gram-positive bacterial infection according to any one of Items 1 to 7, wherein the bacterial infection is by *S. aureus,* and antibiotic-resistant strains thereof, such as MRSA.

Item 9: The pharmaceutical composition according to any one of Items 1 to 8 for use in the prevention and/or treatment of a bacterial infection, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection.

Item 10: The pharmaceutical composition for use according to Item 9, wherein said bacterial infection is caused by a Gram-positive bacterium, preferably wherein said Gram-positive bacterial infection is by *S. aureus,* and antibiotic-resistant strains thereof, such as MRSA.

Item 11. The pharmaceutical composition for use according to Item 9 or 10, wherein said combination of said pharmaceutical composition is administered to a subject simultaneously, separately, or sequentially, wherein said subject is a mammal, such as a human, preferably a human patient, optionally wherein said composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, nasal agent, inhalation mixture, aerosol, mouthwash, mouth spray, nose spray.

Item 12. The pharmaceutical composition for use according to any one of Items 9 to 11, wherein said use furthermore prevents the development and/or spread of bacterial antibiotic resistance.

Item 13: A method for preventing and/or treating a bacterial infection in a subject in need thereof, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection, comprising administering to said subject an effective amount of the pharmaceutical composition according to any one of Items 1 to 8.

Item 14: The method according to Item 13, wherein said bacterial infection is caused by a Gram-positive bacterium, preferably wherein said Gram-positive bacterial infection is by at least one bacterium selected from *S. pneumoniae, B. subtilis, S. aureus,* and antibiotic-resistant strains thereof, such as MRSA.

Item 15: The method according to Item 13 or 14, wherein said combination of said pharmaceutical composition is administered to a subject simultaneously, separately, or sequentially, wherein said subject is a mammal, such as a human, preferably a human patient, optionally wherein said composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray.

Item 16: The method according to any one of Items 13 to 15, wherein said method furthermore prevents the development and/or spread of bacterial antibiotic resistance.

Item 17: A method for producing an antibacterial pharmaceutical composition according to any one of Items 1 to 8, comprising the step of formulating said combination as selected into an antibacterial pharmaceutical composition for the treatment of a Gram-positive bacterial infection.

The invention will now be described further in the following examples with reference to the accompanying figures, nevertheless, without being limited thereto. For the purposes of the present invention, all references as cited are incorporated by reference in their entireties.

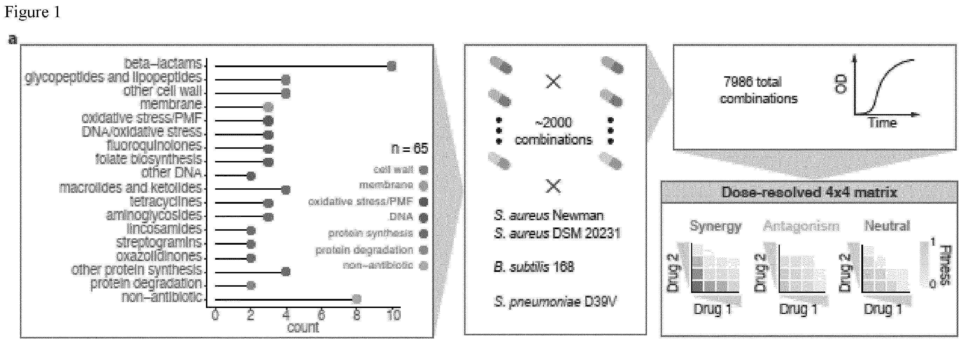

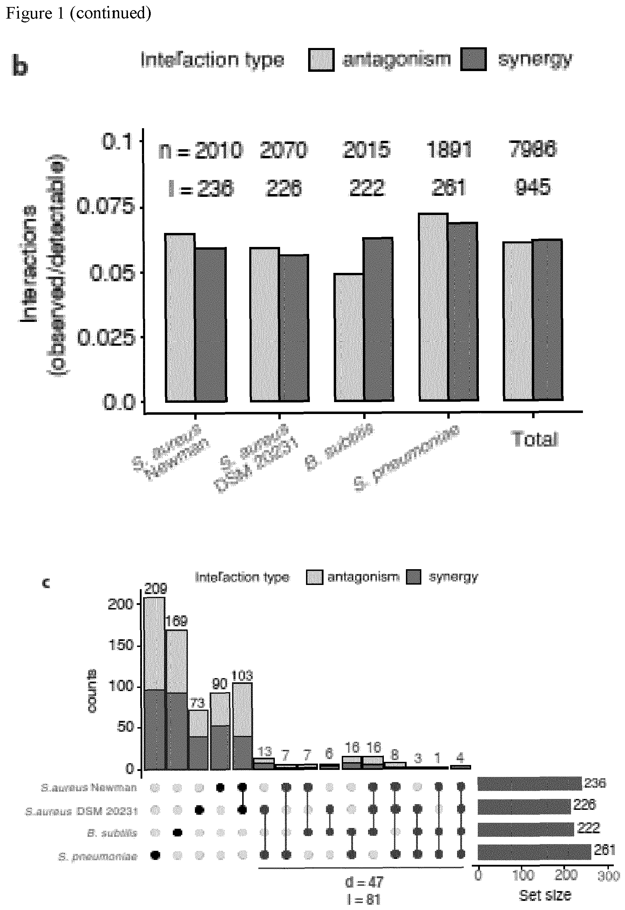

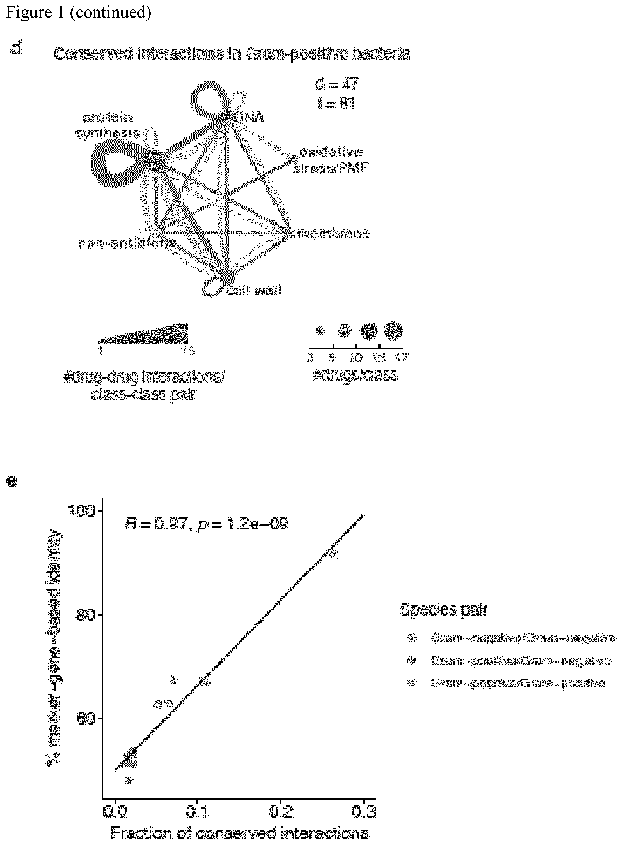

Figure 1 shows that drug-drug interactions are rare and species-specific in Gram-positive bacteria. a. Schematic representation of the high-throughput screen. Pairwise combinations of 65 drugs belonging to several chemical classes and targeting different cellular processes were tested at three concentrations in three bacterial species: *S. aureus* (two strains), *B. subtilis* and *S. pneumoniae.* For each strain, 1891 to 2070 combinations were tested in broth microdilution in 384-well-plates, measuring $OD_{595nm}$ over time. Normalized fitness values were calculated and used to obtain $4 \times 4$ checkerboards and assign interactions as synergistic, antagonistic or neutral (Methods). PMF = proton-motive force. b. Interaction abundance in each strain separately and altogether. Synergy and antagonism frequencies are obtained dividing their absolute counts by the number of combinations for which the probed fitness space allows to detect synergy (fitness upon combination $\geq 0.1$) or antagonism (fitness upon combination $\leq 0.9$) discovery (Methods). Total numbers of combinations tested (n) and detected interactions (i) are shown for each set. c) Conservation of interactions among the four strains tested. All unique interactions detected in the screen (n = 725) are considered to calculate intersection sets between strains. The total number of interactions in each strain is indicated as set size, adding up to 945 total interactions in all strains. The 81 interactions (i) that are conserved across species (involving 47 drugs - d) are highlighted in dark red. d. Network of conserved interactions. Drugs are grouped according to their targeted cellular process. Edge thickness is proportional to the number of drug-drug interactions for each class-class pair. Node size is proportional to the number of drugs in each class. Only drugs involved in this interaction set are considered (d = 47). Nodes are colored according to the targeted cellular processes as in Fig. 1a. e. Drug interaction conservation between species recapitulates phylogeny. Pearson correlation between sequence identity (based on 40 conserved marker genes) and drug interaction conservation rate between pairs of species tested in the context of the present invention and in a previous study (Kavcic, B., Tkacik, G. & Bollenbach, T. Mechanisms of drug interactions between translation-inhibiting antibiotics. Nat. Commun. 11, 4013 (2020)).

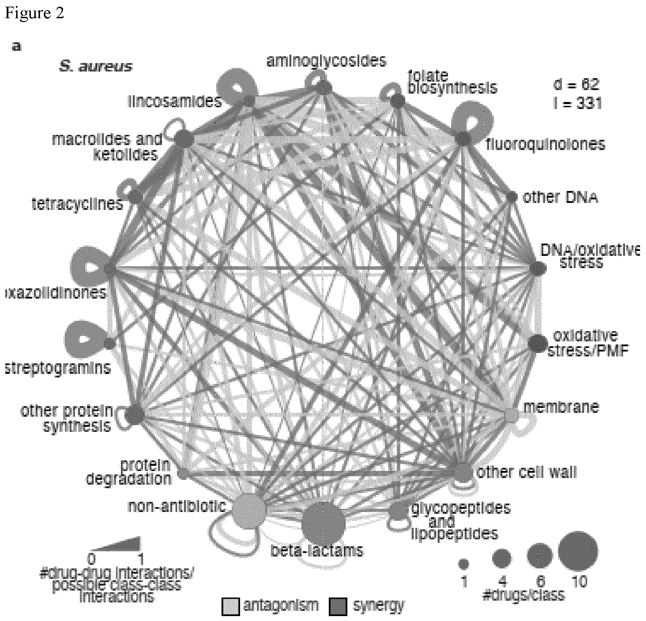

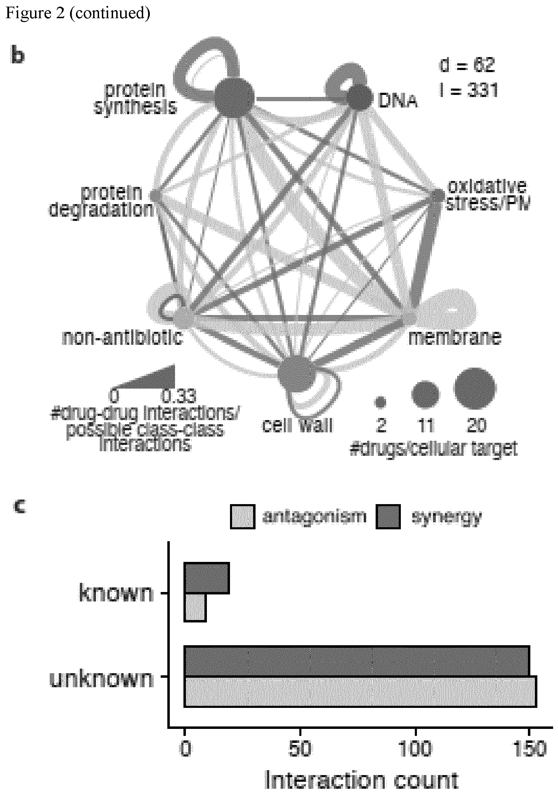

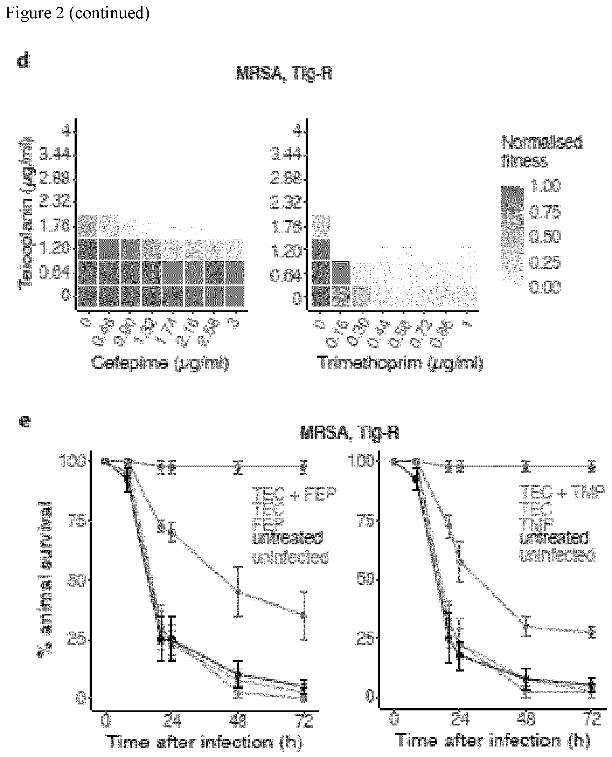

Figure 2 shows that previously uncharacterized synergies in *S. aureus* are active against clinical isolates and in infection models. a-b. Drug interaction networks in S. aureus, with drugs grouped according to their class (a) or targeted cellular process (b). Unique interactions across both strains tested are considered (i = 331). Edge thickness represents the proportion of interactions for each node pair, considering all possible interactions given the number of drugs in each node. Nodes depict the drug class (a) or the targeted cellular process (b), and size is proportional to the number of drugs in the represented class/process. Only interacting drugs are considered (d = 62). Synergies, antagonisms and nodes are colored according to Fig. 1. c. Novel and previously reported interactions detected in S. aureus. Interactions are considered known if reported in any S. aureus strain (Supplementary Table 4). d-e. Novel synergies are effective against MRSA clinical isolates in vitro (d) and in vivo in the G. *mellonella* infection model (e). Teicoplanin (TEC) synergies with cefepime (FEP) and trimethoprim (TMP) were validated against a tigecycline-resistant MRSA clinical isolate in 8 x 8 broth microdilution checkerboards (d) and in G. *mellonella* infection model (e). For checkerboards the median fitness (OD595nm at 7.5h normalized by no-drug controls) across two biological replicates is shown. For G. *mellonella* experiments, larvae were infected with the same MRSA isolate and treated with single drugs or combinations. The percentage of surviving larvae after treatment and in the untreated controls was monitored over time. Uninfected and untreated controls are shown (Methods). Data points represent the mean and error bars indicate standard error (n = 10 for each condition, three independent experiments).

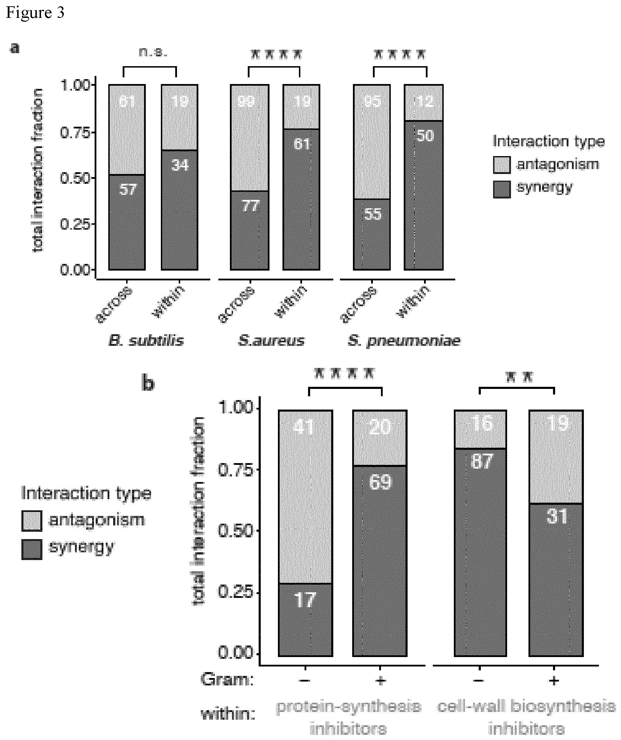

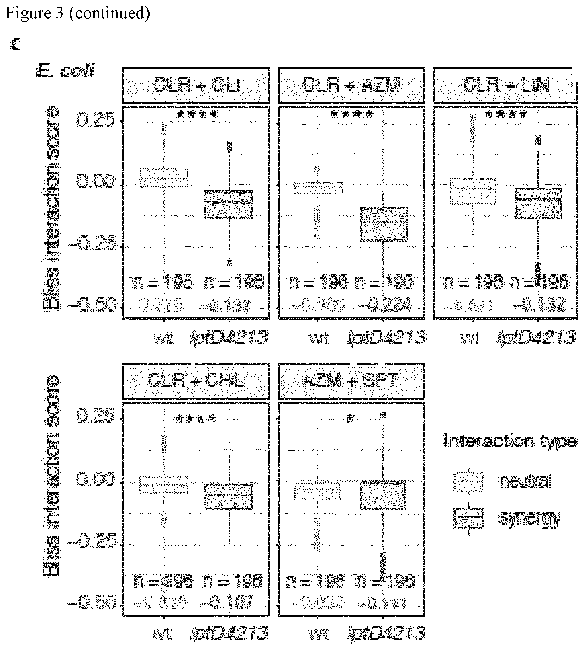

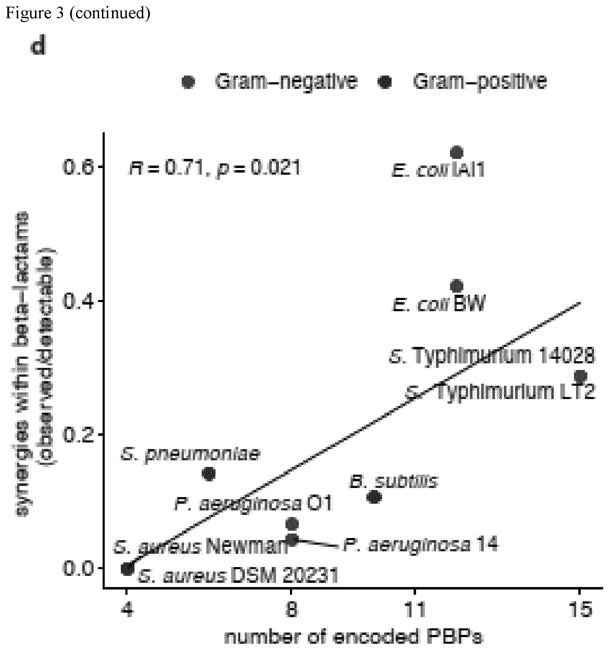

Figure 3 shows distinct synergies between drugs targeting the same cellular process across Gram-positive and -negative species. a. Drugs targeting the same biological process often interact synergistically, whereas antagonisms are prevalent between drugs targeting different processes (*S. aureus:* p = 9.9e-07, $\chi^2$ test; *S. pneumoniae:* p = 4.7e-08, $\chi^2$ test). Non-antibiotic drugs (n = 8) are excluded from this analysis, as their targeted processes are heterogeneous or unknown. b. Gram-positive species exhibit frequent synergistic interactions between protein-synthesis inhibitors, whereas cell-wall biosynthesis inhibitors predominantly synergize in Gram-negative species. Prevalence of interactions between protein-synthesis inhibitors and between cell-wall biosynthesis inhibitors in Gram-negative and Gram-positive species (for protein synthesis inhibitors: p=1.8e-08; for cell-wall biosynthesis inhibitors: p=0.0038, $\chi^2$ test). c. Protein-synthesis inhibitors can also synergize in Gram-negative species when the drug permeability bottleneck is abolished. Synergistic combinations in Gram-positive species were tested in 8 x 8 broth microdilution checkerboards in wild-type E. coli and in the OM-defective *E. coli* lptD4213 strain. Interaction score distributions for each combination are significantly different between the two strains. Interactions were assigned with the same criteria used in the screen, with synergies corresponding to distributions with first quartile < -0.1. The first quartile value is shown in all cases. CLR, clarithromycin; CLI, clindamycin; AZM, azithromycin; LIN, lincomycin; CHL, chloramphenicol, SPT, spectinomycin. d. Differences in beta-lactam synergy prevalence between Gram-negative and Gram-positive species are related to differences in drug target redundancy, that is the Penicillin Binding Proteins (PBPs) they encode in their genomes. Pearson correlation between number of PBPs and the frequency of synergies between beta-lactams for each strain tested.

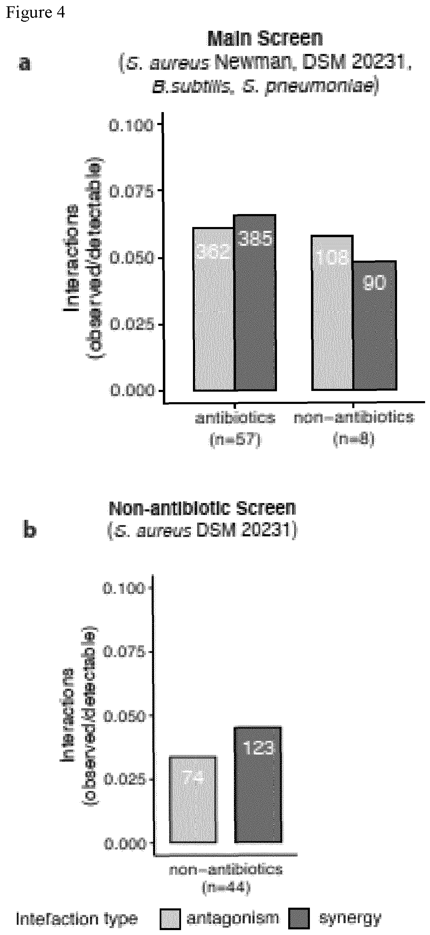

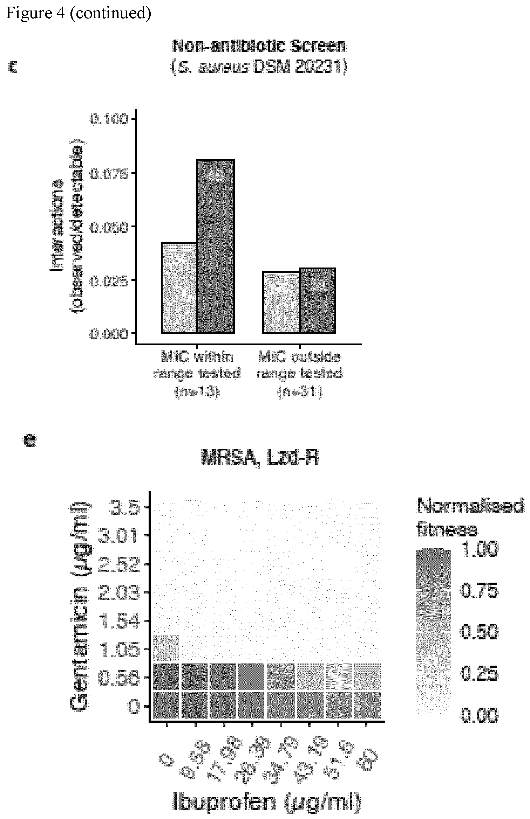

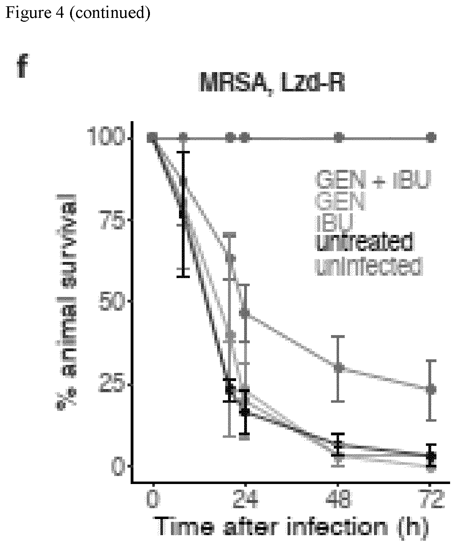

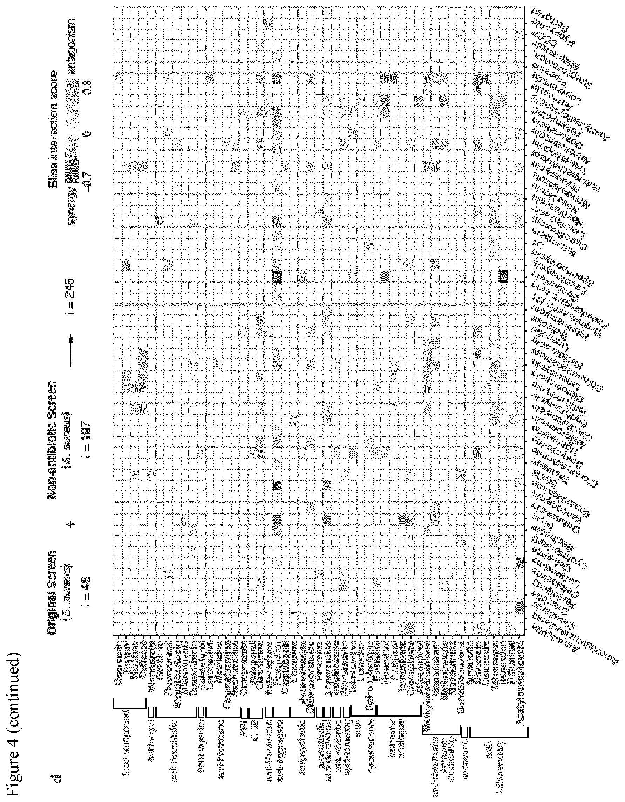

Figure 4 shows interactions between non-antibiotic drugs and antibiotics in S. aureus. a. Interactions of non-antibiotic drugs between themselves and with antibiotics are as common as interactions between two antibiotics. This motivated us to expand the non-antibiotic panel tested. Synergy and antagonism frequencies are calculated as in Fig. 1b. b. Interactions between non-antibiotics and antibiotics in the extended non-antibiotic screen in S. aureus DSM 20231. 44 additional non-antibiotic drugs were screened in combination with 62 drugs belonging to the original drug panel, using the same experimental setup and the same data analysis pipeline, in S. aureus DSM 20231 (Methods). Synergy and antagonism frequencies are calculated as in Figure 1b. c. Non-antibiotics with antibacterial activity, for which the MIC was among tested concentrations (n = 13), engage in more interactions than non-antibiotics for which an MIC does not exist or was not within the tested concentration range (n = 31). d. All interactions between non-antibiotic and antibiotics detected in S. aureus DSM 20231 in the original (i = 87) and in the extended (i = 197) non-antibiotic screen. e-f. The nonsteroidal anti-inflammatory ibuprofen synergizes with gentamicin in MRSA clinical isolates in vitro (e) and in in vivo infection models (f). Gentamicin synergies with ibuprofen and ticagrelor in an MRSA-clinical isolate with additional resistance to linezolid in 8 x 8 broth microdilution checkerboards (e) and in *G. mellonella* infection model (f). Results are obtained and represented as in Fig. 2d-e. GEN, gentamicin; IBU, ibuprofen.

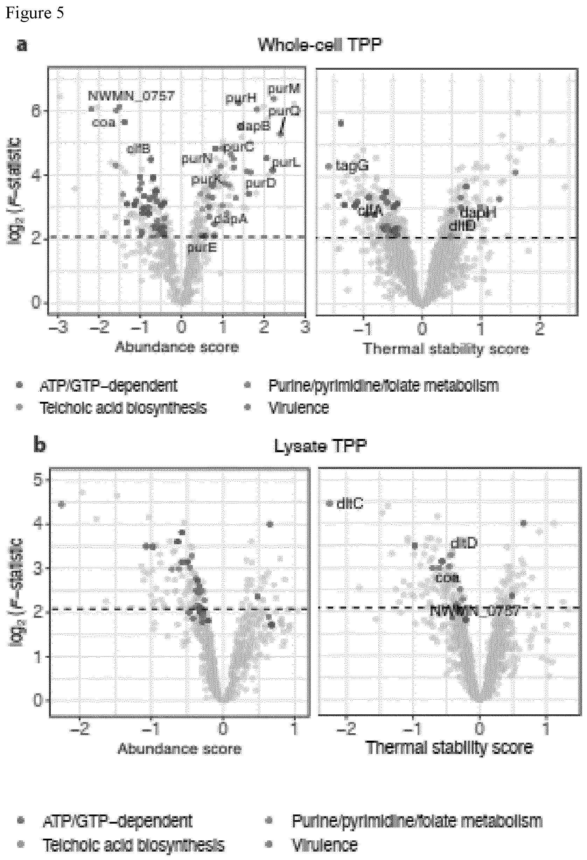

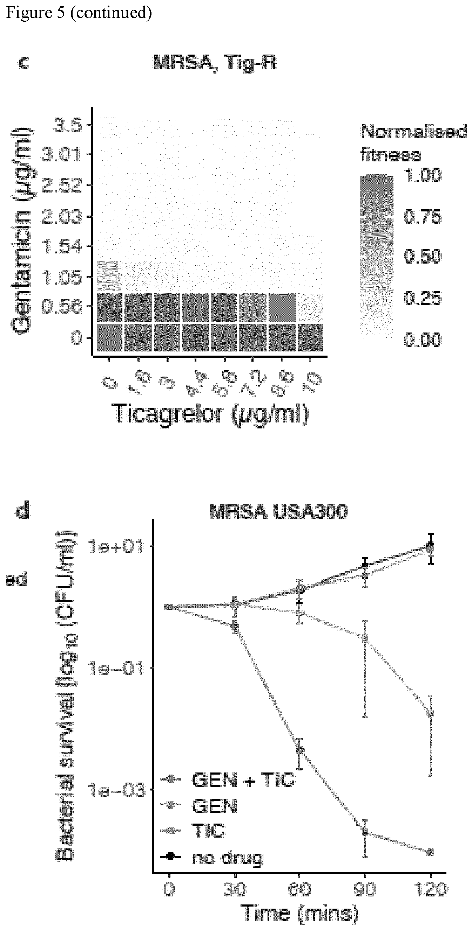

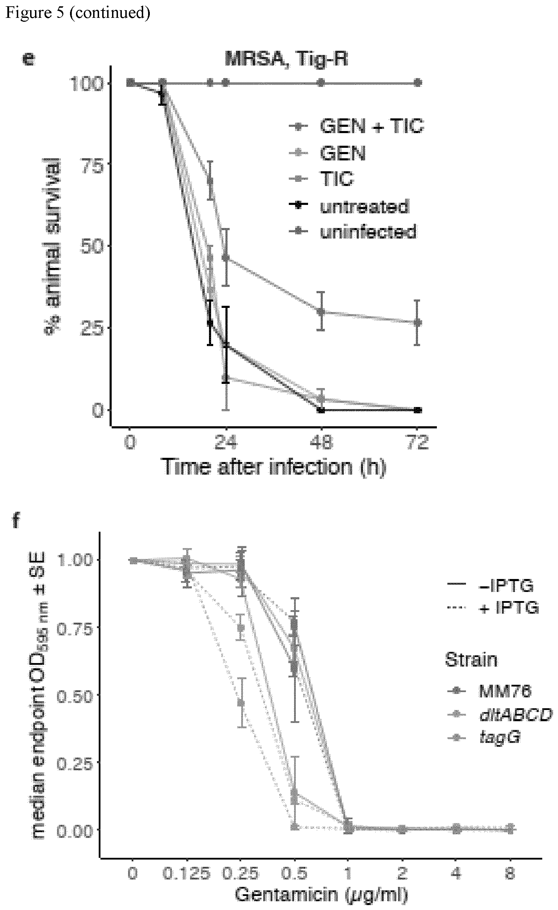

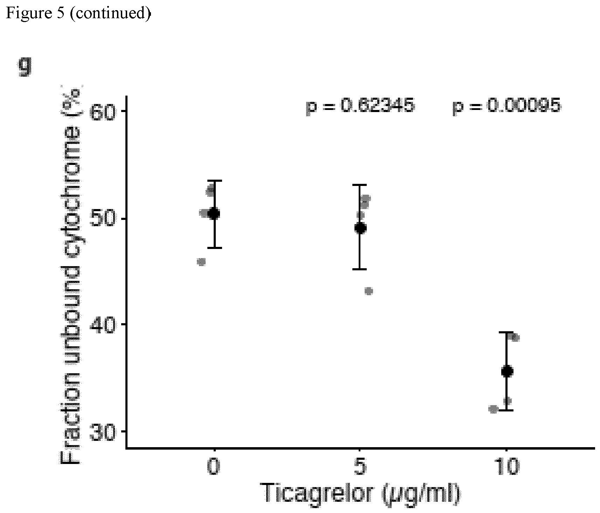

Figure 5 shows that the antiplatelet ticagrelor affects *S. aureus* metabolism and synergizes with cationic antibiotics by altering *S. aureus* surface charge. a-b. Volcano plots highlighting abundance or stability hits in whole-cell (a) and lysate 2D-TPP data (b). The x-axis represents the effect size of protein abundance or stability change as calculated in94 (Methods), and the y-axis corresponds to the statistical significance ($\log_2$(F-statistic). For visualization purposes, when the F-statistic was 0, it was transformed to 1. c-e. Ticagrelor synergizes with gentamicin in vitro at growth inhibition (c;) and killing level (d; mean across four biological replicates; error bars represent standard error) and in vivo (e; mean across ten larvae; error bars represent standard error) against an MRSA isolate resistant to tigecycline. Results for c and e are obtained and represented as described in Fig. 2d-e). GEN, gentamicin; TIC, ticagrelor. f. Growth (endpoint OD595nm, corresponding to the beginning of stationary phase for the control strain MM76, Methods) measured in the presence of serial two-fold dilutions of gentamicin, normalized by no-drug controls, in the *S. aureus* IPTG-inducible knockdown mutants dltABCD and tagG and their control strain MM76 (Methods), in presence or absence of 500 $\mu$M IPTG to induce maximal knockdown of the gene targeted (median across four biological replicates; error bars represent standard error). All strains are grown in presence of 5 $\mu$g/ml erythromycin and 10 $\mu$g/ml chloramphenicol to maintain the CRISPRi plasmid (Methods). g. *S. aureus* Newman surface charge changes upon exposure to ticagrelor. The fraction of positively charged unbound cytochrome C is measured after incubation of drug treated and untreated samples (n = 6, mean and standard error are shown, Methods).

The term "infection", as used in the context of the present invention, relates to the presence of bacteria, viruses, fungi, protozoa or other microorganisms, in or on a subject as well as the invasion by bacteria, viruses, fungi, protozoa or other microorganisms. The invasion includes undesired proliferation of pathogenic microbes in a host organism. More generally, a microbial infection can be any situation in which the presence of a microbial population(s) is damaging to a host animal. Thus, a microbial infection exists when excessive microorganisms are present in or on a mammal's body, or when the effects of the presence of a microbial population(s) is damaging the cells or other tissue of a mammal. Thus, the inhibition of the growth of such invading microorganisms results in a benefit to the subject that is infected by the microbial population(s). Examples of bacterial infections are as mentioned herein.

Said infection to be prevented and/or treated by the compositions of the present invention is preferably caused by a Gram-positive bacterium, wherein preferably said Gram-positive bacterium is a strain of *S. aureus,* optionally wherein said bacterium is an antibiotic-resistant bacterium, such as MRSA, in particular a multi drug resistant strain thereof.

The term "antibiotic" or "antibiotics", as used herein, relates to a chemical substance which at low concentrations kills or prevents the growth of certain microorganisms, generally bacteria, although some antibiotics are also used for the treatment of infections by fungi or protozoa. Antibiotics are used in human, animal or horticultural medicine to treat infections caused by microorganisms. Antibiotics included in the present invention are aminoglycosides, oxazolidinones, streptogramins, carbapenems, cephalosporins, penicillins, glycopeptides, macrolides, ketolides, lantibiotics, quinolones, fluoroquinolones, sulphonamides, tetracyclines, glycylcyclines, and others such as vancomycin, daptomycin, trimethoprim, novobiocin, chloramphenicol, clindamycin, lincomycin, fosfomycin, fusidic acid, metronidazole, mupirocin, nitrofurantoin, quinupristin/dalfopristin, or a rifamycin, such as rifampicin.

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection, and infusion. Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art. Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, in one embodiment the carrier is an isotonic buffered saline solution. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. A typical dose can be, for example, in the range of 0.001 to 1000 μg; however, doses below or above this exemplary range are envisioned, especially considering the aforementioned factors. Generally, the regimen as a regular administration of the pharmaceutical composition should be in the range of 1 μg to 10 mg units per day. If the regimen is a continuous infusion, it should also be in the range of 1 μg to 10 mg units per kilogram of body weight per minute, respectively. Progress can be monitored by periodic assessment. The compositions of the invention may be administered locally or systemically. Administration will generally be parenterally, e.g., intravenously; the pharmaceutical composition may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media.

Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins or interferons depending on the intended use of the pharmaceutical composition.

In context of the present invention, the term "subject", as used in certain embodiments, preferably refers to a mammal, such as a mouse, rat, guinea pig, rabbit, cat, dog, monkey, or preferably a human, and a patient. The term "patient" preferably refers to a mammal, such as a mouse, rat, guinea pig, rabbit, horse, cattle, cow, cat, dog, monkey, or preferably a human, for example a human patient, for whom diagnosis, prognosis, or therapy is desired. The subject of the invention may be at danger of suffering from a disease, such as a bacterial infection, a viral infection, a fungal infection, and a parasitic infection. A more detailed description of medical indications relevant in context of the invention is provided herein.

The term "treating" as used herein means stabilizing or reducing an adverse symptom associated with a condition; reducing the severity of a disease symptom; slowing the rate of the progression of a disease; inhibiting or stabilizing the progression of a disease condition; or changing a metric that is associated with the disease state in a desirable way.

By synergy, the inventors define an antimicrobial effect (quantified as growth in broth microdilution assay or survival of infected hosts) greater than either drug alone and more what expected according to the neutrality assumption of the Bliss interaction model (Bliss, C. I. THE TOXICITY OF POISONS APPLIED JOINTLY. Ann. Appl. Biol. 26, 585-822 615 (1939)), i.e. the multiplication of the single-drug effects as also described herein.

## Examples

The inventors present a systematic and quantitative account of drug interactions in key Gram-positive species, discovering a number of potent synergies that are effective against clinical MDR isolates, and providing insights into the underlying mechanisms of some of the observed interactions. In an era where novel antibiotic development faces technical and economic hurdles, and new antimicrobial strategies are urgently needed, systematic drug interaction profiling can offer possible solutions to treat bacterial infections. Extending the systematic testing of drug interactions to more bacteria and to other types of non-antibiotic drugs improves our understanding of drug interaction conservation and mechanisms, and inform tailored treatments towards pathogens.

In the context of the present invention, synergies were identified by means of a high-throughput drug-drug interaction screen performed in *Staphylococcus aureus* lab strains, validated in a high-resolved, low-throughput setup *in vitro* and confirmed *in vivo* as effective against infection by MRSA clinical isolates in the greater wax moth *Galleria mellonella*.

## Methods

### Strains and growth conditions

*B. subtilis subsp subtilis* 168 was kindly provided by Carol A. Gross, all MRSA clinical isolates by Stephan Göttig, *S. pneumoniae* D39V75 by Jan-Willem Veening, and S. aureus USA300 by Daniel Lopez. *Staphylococcus aureus subsp aureus* Newman was purchased from NCTC (NCTC 8178). The type strain *Staphylococcus aureus subsp aureus* DSM 2023177 (ATCC 12600 T, NCTC 8532) was purchased from DSMZ, Braunschweig, Germany. For all experiments and unless otherwise specified, *S. aureus* strains were grown in Tryptic Soy Broth (TSB, ref. 22092 by Merck-Millipore), B. subtilis was grown in LB Lennox, and *S. pneumoniae* was grown in CY medium, adapted from (Martin B, Garcia P, Castanié M-P, Claverys J-P. The recA gene of Streptococcus pneumoniae is part of a competence-induced operon and controls lysogenic induction. Molecular Microbiology 15, 367-379 (1995)). All species were grown at 37°C, with vigorous shaking, except for *S. pneumoniae* (without shaking). The ticagrelor purine supplementation experiments in *S. aureus* Newman were conducted in SSM9PR defined medium supplemented with 1% glucose.

### Inducible knockdown strain construction

A two-plasmid CRISPR interference system was used to knock down gene expression of selected genes in *S. aureus* Newman. In these strains, dcas is expressed from an IPTG-inducible promoter on plasmid pLOW, while sgRNAs are expressed from a constitute promoter on a plasmid derived from pCG248. The sgRNA-target sequences were TGTCTAACAGCAATGCTTTG (SEQ ID NO. 1) for dltABCD and AAACCATAATTTGCATAACA (SEQ ID NO. 2) for tagG, and ATAGAGGATAGAATGGCGCC (SEQ ID NO. 3) for the non-target control MM76.

**MIC determination**

MICs were tested in all strains for the main screen. Drugs were two-fold serially diluted in 11 concentrations, and 32 no-drug control wells were included in each plate. Experiments were conducted in 384-well plates (ref. 781271 by Greiner BioOne), with a total volume of 30 $\mu$l for *S. aureus* and *B. subtilis* and 55 $\mu$l for *S. pneumoniae.* Volumes were optimized for each strain to achieve good dynamic range for growth and minimize risk of cross-contamination between wells. Plates were inoculated with a starting optical density at 595 nm ($OD_{595nm}$) of 0.01 from an overnight culture. All liquid handling was performed using a Biomek FX liquid handler (Beckman Coulter). Plates were sealed with breathable membranes and incubated at 37°C. $OD_{595nm}$ was measured every 30 minutes for 14 hours. The MIC was considered as the first concentration at which growth was inhibited. Experiments were conducted in biological duplicates. For the adjuvant screen, antibiotics were tested in *S. aureus* DSM 20231 at the same concentrations as for the main screen. Non-antibiotics concentrations were selected to fall within therapeutic ranges.

**High-throughput screen of drug combinations**

62 drugs, hereafter designated as recipients, were arrayed in 384-well plates (ref. 781271 by Greiner BioOne) in three two-fold serial dilutions and two technical replicates. Concentrations were selected according to MICs, with the highest concentration corresponding to the MIC, and the intermediate and lowest concentration corresponding to ½ and ¼ MIC, respectively. Plates were kept frozen and defrosted upon each experimental run, when the same 62 drugs and in the same three concentrations were added as donor drugs (one drug at one concentration for each recipient plate). A few drugs were screened only as donors: the combinations co-amoxiclav and cotrimoxazole in B. *subtilis* and *S. aureus* DSM 20231; co-amoxiclav, clavulanic acid, pseudomonic acid and cefuroxime in *S. aureus* Newman. All donor drugs were tested in two biological replicates. Control wells were included in each plate (six no-drug wells, three plain medium wells and three wells containing only the donor drug). After the addition of donor drugs, plates were inoculated with cells. Handling, inoculation, growth conditions, plate incubation and OD595nm measurements were performed as for the MIC determination.

For the adjuvant screen, 44 non-antibiotic drugs were tested against the same recipient drugs of the main screen for *S. aureus* DSM 20231.

**Screen benchmarking and 8 × 8 checkerboard assays**

Combinations were tested in the same experimental conditions as for the screen, but at higher concentration resolution: drugs were diluted in eight concentrations spanning linearly-space gradients to assemble 8 × 8 checkerboards for each combination tested. The highest concentration corresponded to the highest concentration in the screen, or slightly lower to ensure better mapping of the dose-response space from full to no growth inhibition. All experiments were conducted in at least two technical and two biological replicates. Data was analyzed with the same pipeline as for the screen (Methods).

**Data analysis**

The data analysis was adapted from (Brochado, A. R. et al. Species-specific activity of antibacterial drug combinations. Nature 559, 259-263 (2018)). Growth curves were processed as described in and the background was subtracted from all OD595nm measurements, on a well-by-well basis using the first measurement obtained. Abnormal spikes in OD values of the first three time points occurred in *S. pneumoniae* in a small fraction of wells due to bubble formation in the medium or plate condensation. These early local peaks in OD curves were identified and replaced with the median of OD values (of corresponding time points) estimated from the wells not affected by such artefacts within the same plate. When more than one in the first four time points was affected, those wells were identified as non-monotonically-increasing $OD_{595nm}$ values across the first four time points, and their background was estimated as the median first-time-point OD595nm of artefact-free wells (monotonically increasing across the first four time points).

The time point corresponding to the transition between exponential and stationary phase in no-drug control wells was identified as the first time point at which the maximum OD595nm was reached. This time point was selected according to the growth characteristics of each strain: 8 h for both *S. aureus* strains, 5.5 h for *B. subtilis* and 3.7 h for *S. pneumoniae.* Later time points were excluded from further analysis. The OD595nm measurement at that time point was used to derive fitness measurements that captured effects both on growth rate and maximum yield. OD-based endpoints correlated well with AUC-based endpoints (Pearson correlation 0.95), and led to higher precision and recall according to the screen benchmarking. This value was then divided, per plate, by the robust mean (Huber, P. J. Robust Estimation of a Location Parameter. Ann. Math. Stat. 35, 73-101 (1964)) of the six no-drug controls (no-drug control hereafter), obtaining three fitness measures for each drug concentration pair: f1, fitness upon exposure to drug 1; f2, fitness upon exposure to drug 2; and f1,2, fitness in presence of drug 1 + drug 2.

Based on these values, further quality control was again performed, correcting fitness increase artefacts (maximum fitness was set to 1) and removing plates with poor technical replicate correlation (Pearson correlation < 0.7). f1, f2, and f1,2 were used to calculate interaction scores using the Bliss model (Bliss, C. I. THE TOXICITY OF POISONS APPLIED JOINTLY. Ann. Appl. Biol. 26, 585-615 (1939)). The choice of this model over other available quantification methods was driven by the following considerations: (i) the three measurements obtained for drug dose responses are not sufficient for accurate quantification using alternative models (e.g. the Loewe model (Goldoni, M. & Johansson, C. A mathematical approach to study combined effects of toxicants in vitro: evaluation of the Bliss independence criterion and the Loewe additivity model. Toxicol. In Vitro 21, 759-769 (2007)) and (ii) the Bliss model can more accurately account for single drugs with no effect (such as most non-antibiotic drugs included in the screen).

Bliss (e) scores were calculated as follows:

$$\varepsilon = f_{d1,d2} - f_{d1} * f_{d2}$$

[eq.1]

where fdld2 corresponds to the observed fitness in the presence of the drug combination, and fd1 and fd2 correspond to the fitness in the presence of the two single drugs.

Single drug fitness for both donor and recipient drugs can also be inferred from combination fitness, by minimizing the sum of residuals squared of the Bliss independence model as follows and using the assumption that most drugs interact neutrally:

$$\{f_{d1}, f_{d2}\} = \arg\min \sum_{d1,d2} \|f_{d1,d2} - f_{d1} * f_{d2}\|^2$$

[eq.2]

Experimentally measured and estimated fitness values were very similar for donor and recipient drugs, and the inventors used the estimated measures since those were more robust to noise - experimental controls were limited for donor drugs (three single-drug control wells) and sometimes biased for recipient drugs, as a single problematic plate in the batch was sufficient to generate noise.

When no data was discarded upon quality control, the number of Bliss scores obtained for each combination was 72, composed of 3 x 3 (in the two-dimensional concentration space) x two technical replicates x two biological replicates x two replicates with drugs tested as donor or recipient. Hit calling was performed using a resampling procedure with 10,000 repetitions for each combination tested, where the e distribution for each combination was compared with the resampled Bliss scores using Wilcoxon rank-sum test in each iteration. Hits correspond to combinations with FDR < 0.05.

As before (Brochado, A. R. et al. Species-specific activity of antibacterial drug combinations. Nature 559, 259-263 (2018)), the inventors coupled this significance threshold to an effect-size threshold. For each combination the inventors defined a cumulative score using the quartiles of its distribution of e scores. The inventors tested the performance of different thresholds in precision and recall upon screen benchmarking, and identified |0.1| as best threshold, with precision 0.87 and recall 0.68. Accordingly, synergies were assigned if the first quartile of the ε distribution < -0.1 and antagonisms if the third quartile exceeded 0.1. The inventors could increase the screen recall by leveraging the presence of two strains belonging to the same species in the case of S. *aureus,* as previously described for Gram-negative species (Brochado, A. R. et al. Species-specific activity of antibacterial drug combinations. Nature 559, 259-263 (2018)). The inventors defined an additional set of hits (weak and conserved), meeting significance and effect size thresholds in one strain, but with lower effect size in the other strain. A cutoff of |0.08| allowed to maintain the same precision and increase the recall to 0.72.

Data analysis was implemented with R v.4.1.283 and RStudio v.2021.09.184 and networks were created with Cytoscape v.3.8.285.

### Interaction detection calculation

Interaction detection rates were calculated by dividing the number of detected interactions by the number of combinations for which interactions could be observed according to the mapped fitness space. Synergies could not be observed when the expected fitness of a drug combination (defined as the product of single-drug fitness values in eq. 1) was lower than 0.1, while antagonisms could not be detected for expected combination fitness higher than 0.9 (2.1 and 3.3% of the 7986 combinations tested, respectively).

**Drug clustering**

Drug-drug interaction profiles were clustered according to the cosine similarity of quartile-based Bliss interaction scores of each drug pair in each strain. Scores from all interactions were considered, regardless of their statistical significance. For the clustering based on chemical structures, drugs were clustered according to their Tanimoto similarity (Bajusz, D., Racz, A. & Héberger, K. Why is Tanimoto index an appropriate choice for fingerprint-based similarity calculations? J. Cheminform. 7, 20 (2015)) using 1024-bit ECFP4 fingerprints (Morgan, H. L. The Generation of a Unique Machine Description for Chemical Structures-A Technique Developed at Chemical Abstracts Service. J. Chem. Doc. 5, 107-113 (1965)).

**Phylogeny analysis**

To calculate the percentage sequence identity between bacterial species, the genomes of *B. subtilis* 168, *S. aureus* Newman, *S. aureus* DSM 20231, *S. pneumoniae* D39, *E. coli* K-12, *S. enterica* serovar Typhimurium LT and *P. aeruginosa* PAO1 were downloaded from NCBI and 40 universal single-copy marker genes (MGs) were extracted using the fetchMG script. The 40 MGs were selected from a previous publication for their ability to characterize prokaryotic species, and they encode for ubiquitous functions like tRNA synthetases or are ribosomal proteins (EggNOG COGs: COG0012, COG0016, COG0018, COG0048, COG0049, COG0052, COG0080, COG0081, COG0085, COG0087, COG0088, COG0090, COG0091, COG0092, COG0093, COG0094, COG0096, COG0097, COG0098, COG0099, COG0100, COG0102, COG0103, COG0124, COG0172, COG0184, COG0185, COG0186, COG0197, COG0200, COG0201, COG0202, COG0215, COG0256, COG0495, COG0522, COG0525, COG0533, COG0541, COG0552). The concatenated sequences (all six genomes contained exactly 40 MGs) were used to calculate percentage nucleotide sequence identity with vsearch (Rognes, T., Flouri, T., Nichols, B., Quince, C. & Mahe, F. VSEARCH: a versatile open-source tool for metagenomics. PeerJ 4, e2584 (2016)) and to create a phylogenetic tree. To this end, a multiple sequence alignment was created with muscle v3.8.155190 with default parameters. Finally, a maximum-likelihood phylogenetic tree was constructed using the online tool PhymL 3.091 with default parameters. To evaluate interaction conservation, only the 46 drugs tested both in Gram-positive and Gram-negative species were considered.

**Evaluation of drug combination therapy using the *G. mellonella* infection model**

Larvae of the greater wax moth (*Galleria mellonella*) at their final instar larval stage were used for evaluation of selected drug combinations to assess their efficacy against MRSA in vivo. Larvae were purchased from UK Waxworms (Sheffield, UK) and Mucha Terra (Ahaus-Altstätte, Germany). Stock solutions of cefepime, gentamicin, ibuprofen, teicoplanin and trimethoprim were freshly prepared as described for the *in vitro* experiments with the exception of ticagrelor which was dissolved in 50 mM EtOH and diluted in distilled water to the required concentration. The MRSA strains were cultivated in brain heart infusion medium and harvested at an OD600 of 0.5. Bacteria were washed twice with PBS and adjusted to an OD600 which corresponded to a lethal dose of approximately 75% (LD75) of the larvae after 24 h (approximately $10^7$ CFUs). Ten larvae per condition were injected with 10 µL of the bacterial cell suspension or PBS (referred to as uninfected control) into the hemocoel via the last left proleg using Hamilton precision syringes. After one hour, 10 µL of single drugs or combinations were injected into the last right proleg, at the following drug concentrations: teicoplanin 1 µg/ml, trimethoprim 250 µg/ml, cefepime 0.025 µg/ml, gentamicin 2 µg/ml, ibuprofen 4 µg/ml, ticagrelor 100 µg/ml. The survival of *Galleria* larvae was monitored at the indicated time points by two observers independently. Each strain-drug combination was evaluated in three independent experiments.

**Time-kill experiments**

Overnight cultures of *S. aureus* USA300 were diluted 1:100 in 20 ml of TSB medium, incubated for 1h in flasks at 37°C with continuous shaking and diluted again 1:100 in 20 ml prewarmed TSB with ticagrelor (5 µg/ml), gentamicin (1.5 µg/ml), their combination or without drugs. 50 µl of serial 10-fold dilutions of cultures were plated on TSA plates every 30 minutes for 2 hours. Cell viability was determined by counting CFUs after plates were incubated overnight in four independent experiments.

**Two-dimensional thermal proteome profiling (2D-TPP)**

Bacterial cells were grown overnight at 37°C in TSB and diluted 1000-fold into 50 ml of fresh medium. Cultures were grown at 37°C with shaking until OD578 ~0.6. Ticagrelor at the desired concentrations (0.04, 0.16, 0.8 and 4 µg/ml) or a vehicle-treated control were added and cultures were incubated at 37°C for 10 minutes. Cells were then pelleted at 4,000 x g for 5 min, washed with 10 ml PBS containing the drug at the appropriate concentrations, resuspended in the same buffer to an OD578 of 10 and aliquoted to a PCR plate. The plate was then exposed to a temperature gradient for 3 min in a PCR machine (Agilent SureCycler 8800), followed by 3 min at room temperature. Cells were lysed with lysis buffer (final concentration: 50 $\mu$g/ml lysostaphin, 0.8% NP-40, 1x protease inhibitor (Roche), 250 U/ml benzonase and 1 mM MgCl2 in PBS) for 20 min, shaking at room temperature, followed by five freeze-thaw cycles. Protein aggregates were then removed by centrifuging the plate at 2,000 x g and filtering the supernatant at 500 x g through a 0.45 $\mu$m filter plate for 5 minutes at 4°C. Protein digestion, peptide labelling, and MS-based proteomics were performed as previously described (Mateus, A. et al. Thermal proteome profiling in bacteria: probing protein state in vivo. Mol. Syst. Biol. 14, e8242 (2018)).

**2D-TPP data analysis**

Data were pre-processed and normalized as previously described (Becher, I. et al. Thermal profiling reveals phenylalanine hydroxylase as an off-target of panobinostat. Nat. Chem. Biol. 12, 908-910 (2016)). Peptide and protein identification were performed against the *S. aureus* Newman strain Uniprot FASTA (Proteome ID: UP000006386), modified to include known contaminants and the reversed protein sequences. Data analysis was performed in R using the package TPP2D92 as previously described (Kurzawa, N. et al. Computational analysis of ligand dose range thermal proteome profiles. 2020.05.08.083709 (2020) doi:10.1101/2020.05.08.083709.). Briefly, to identify stability changes, a null model, allowing the soluble protein fraction to depend only on temperature, and an alternative model, corresponding to a sigmoidal dose-response function for each temperature step, are fitted to the data. For each protein the residual sum of squares (RSS) of the two models are compared to obtain an F-statistic. FDR control is performed with a bootstrap procedure as previously described (Kurzawa, N. et al. Computational analysis of ligand dose range thermal proteome profiles. 2020.05.08.083709 (2020) doi:10.1101/2020.05.08.083709). The abundance or thermal stability effect size was calculated for each protein as following:

$$\text{sign}(\kappa) \cdot \sqrt{RSS^0 - RSS^1}$$

[eq.3]

where k is the slope of the dose-response model fitted across temperatures and drug concentrations and RSS0 and RSS1 correspond to the residual sum of squares of the null (pEC50 linearly scaling with temperature) and alternative model, respectively.

**KEGG enrichment**

*S. aureus* Newman proteome was annotated using KEGG95 (release 100.0, October 1). Proteins with missing KEGG annotation were preliminarily removed. Fisher's exact test was then used to test the enrichment of input protein sets (hits corresponding to FDR < 0.05) against the background (all detected proteins) for each term. The p-values were corrected for multiple testing using the Benjamini-Hochberg procedure. The analysis was performed in R using the packages KEGGREST (Tenenbaum D, M. B. KEGGREST: Client-side REST access to the Kyoto Encyclopedia of Genes and Genomes (KEGG). R package version 1.34.0. (2021)), EnrichmentBrowser (Geistlinger, L., Csaba, G. & Zimmer, R. Bioconductor's EnrichmentBrowser: seamless navigation through combined results of set- & network-based enrichment analysis. BMC Bioinformatics 17, 45 (2016)) and clusterProfiler (Wu, T. et al. clusterProfiler 4.0: A universal enrichment tool for interpreting omics data. Innovation (N Y) 2, 100141 (2021)).

**Ticagrelor MIC upon purine depletion and supplementation**

Ticagrelor (ref. SML2482, Sigma-Aldrich) MIC was measured upon purine supplementation in S. aureus Newman as described above in SSM9PR minimal medium supplemented with 1% glucose79, in 384-well plates (ref. 781271 by Greiner BioOne) with a final volume of 30 $\mu$l. Adenine and inosine were added at 20 and 100 $\mu$g/ml or in combination, both at 100 $\mu$g/ml. Experiments were conducted in four biological replicates. A single time-point OD595nm at the transition between exponential and stationary phase (13.5 h) was used to derive dose-response curves, after normalization to the respective no-drug control for each condition.

**Gentamicin and nisin MIC measurements in dltABCD and tagG knockdown mutants**

For gentamicin and nisin MIC measurements, dltABCD and tagG IPTG-inducible knockdown mutants (Methods) were grown in two-fold dilutions of nisin and gentamicin, in presence of erythromycin (5 $\mu$g/ml) and chloramphenicol (10$\mu$g/ml) for plasmid maintenance. IPTG (500 $\mu$M) was used to achieve maximal knockdown of the gene targeted. The parent *S. aureus* Newman and the control strain MM76 (Methods) were included in all experiments, and experiments were conducted in four biological replicates, in 384-well plates (ref. 781271 by Greiner BioOne). For each plate, the inventors identified the time point where the control strain MM76 (in presence of erythromycin, chloramphenicol and IPTG at the above-mentioned concentrations) reached plateau, defined as the first time point before no increase was detected in log10 (OD595nm) values of two consecutive time points. This time point was then used for all wells to derive dose-response curves, after normalization to the respective no-drug control for each strain and biological replicate.

**Determination of cell surface charge**

The cytochrome c binding assay was conducted as previously described (Radlinski, L. C. et al. Chemical Induction of Aminoglycoside Uptake Overcomes Antibiotic Tolerance and Resistance in Staphylococcus aureus. Cell Chem Biol 26, 1355-1364.e4 (2019)). Briefly, overnight cultures of *S. aureus* Newman were diluted 1:1000 in 20 ml of TSB medium and grown in flasks at 37°C with continuous shaking until they reached OD578nm ~ 0.45. Samples were then incubated in the same conditions with or without 10 and 5 $\mu$g/ml ticagrelor for 20 minutes. Samples were centrifuged at 10000 g for 15 minutes, washed twice with 20 mM MOPS buffer (pH 7) and concentrated to reach a final A578 of 10 in a 96 well-plate (ref. 4483481, Applied Biosystems™) containing cytochrome c (0.25 mg/ml, ref. 101467, MP Bio) or MOPS buffer (Fig. 5g). The plate was incubated in the dark at room temperature for 10 minutes. The cell pellets were collected, and the amount of cytochrome c in the supernatant was determined spectrophotometrically at an OD410nm. Two-fold dilutions of cytochrome c in the same plate, starting from 256 $\mu$g/ml, were used to obtain a standard curve onto which a linear model was fitted to calculate cytochrome c concentrations in the other wells. Results are expressed as unbound cytochrome c fraction in the supernatants. Experiments were conducted in four biological replicates.

**Results**

The inventors profiled 7986 drug combinations in a 4 x 4 dose matrix (two-fold dilution gradient) in *Staphylococcus aureus, Streptococcus pneumoniae* and *Bacillus subtilis.* For *S. aureus,* two strains (*S. aureus* Newman and DSM 20231) were probed to assess within-species conservation (Fig. 1a). The drug panel (n = 65) included most antibiotics (n = 57) used against infections with Gram-positive bacteria, belonging to all main classes and targeting different bacterial processes, and eight other bioactive molecules, such as antifungals, drugs with human targets and food additives - depicted as non-antibiotics.

The inventors measured growth in a broth microdilution format in microtiter plates using optical density (OD595nm) as a readout. Media and shaking conditions were different for each species (Methods). Drug concentrations were tailored after measuring minimal inhibitory concentrations (MICs) for each drug in the four strains, with the highest concentration corresponding to the MIC in most cases, and the intermediate and lowest concentration corresponding to 1/2 and 1/4 of the highest concentration, respectively (Methods). The inventors derived relative fitness values in the presence of single drugs and drug combinations, dividing single-time point OD595nm values upon drug treatment by the corresponding values of no-drug controls at the same time point. This time point was selected according to the growth characteristics of each strain and aimed to capture both growth rate and yield effects (Methods). The inventors conducted all experiments in biological (i.e. overnight cultures obtained from distinct colonies) and technical (i.e. replicated wells in the same plate) duplicates, achieving high replicate correlations (average Pearson correlation 0.84-0.89 for biological and 0.91 for technical replicates. Single time-point OD- and area under the growth-curve (AUC)-based fitness values led to very similar results, with the former being more robust. Similarly, estimated single-drug fitness values mirrored experimentally-measured values and were further used since they were derived from more measurements. From the 4 x 4 concentration matrices of relative fitness values, the inventors calculated interaction scores using the Bliss interaction model (Methods). For each pairwise combination, a cumulative effect size value was derived from the distribution of interaction scores, consisting of at least 72 interaction scores including all replicates of individual concentration combinations for each drug pair. The first and third quartile values of this distribution were taken as cumulative effect size values for synergies and antagonisms, respectively, with negative values corresponding to synergies and positive values to antagonisms (Methods).

To calibrate hit scoring, as well as to assess the high-throughput screen data quality, the inventors benchmarked the screen data against a validation set of 161 combinations (2% of screened combinations), equally representing the four strains probed. These combinations were tested in the same growth conditions as the inventors' high-throughput screen, but over a highly-resolved dose space (8 × 8 matrix) of linearly-spaced concentration dilution gradients (Methods), and including hits and neutral interactions to about equal parts. The precision-recall curves were comparable to the previous Gram-negative screen (Brochado, A. R. et al. Species-specific activity of antibacterial drug combinations. Nature 559, 259-263 (2018)), with highest precision (0.87) and recall (0.68) observed for a threshold on absolute effect size of > 0.1 and on Benjamini-Hochberg adjusted p-value of < 0.05 (resampling procedure with 10,000 repetitions for each combination tested, comparison with resampled Bliss scores using Wilcoxon rank-sum test in each iteration) (Methods). The inventors were able to slightly increase the recall (0.72) by relaxing the effect size thresholds for interactions found in both *S. aureus* strains, using within-species conservation as an additional parameter to confirm interactions (Methods).

**Drug interactions are rare and species-specific**

Antagonisms and synergies were about equally prevalent across the three species, accounting for ~12% of all combinations tested (Fig. 1b). This interaction rate, corrected by the inventors' ability to observe synergies or antagonisms according to the fitness space probed for each combination (Methods), is lower and less skewed towards antagonisms as compared to Gram-negative species (*E. coli, S. Typhimurium, P. aeruginosa*). This could be due to technical reasons: drug or strain selection biases, and/or the fact that only one strain was tested for *B. subtilis* and *S. pneumoniae* species, thus preventing the use of within-species conservation as a criterion to increase recall as for all Gram-negative species. Alternatively, differences could be driven by biological reasons. For example, Gram-positive bacteria have a lower drug permeability bottleneck than Gram-negative bacteria, owing to the absence of an outer membrane. As a consequence, interactions relying on drug permeability modulation may be less frequent in Gram-positive bacteria. In contrast, Gram-negative species show a high prevalence of antagonisms because many interactions depend on intracellular drug availability. In support of this hypothesis, the overall synergy rate was only marginally lower than in Gram-negative species (6.2% and 7.3%, respectively), whereas antagonisms were rarer in Gram-positive bacteria (6%, as compared to 10.6% rate in Gram-negative species).

As an example, in Gram-positive species the inventors only found 4 out of the 10 antagonisms confirmed to be dependent on drug concentration modulation in Gram-negative bacteria. Overall, this suggests that in Gram-positive bacteria drug antagonisms are rarer, likely due to the lower drug permeability barrier than that of Gram-negative bacteria.

Species-specificity of drug interactions has long been assumed (Jawetz, E. The use of combinations of antimicrobial drugs. Annu. Rev. Pharmacol. 8, 151-170 (1968)), and recently systematically demonstrated for Gram-negative species, with 30% of detected interactions shared between at least two of the three species tested, and 5% conserved in all three species (*E. coli, S. typhimurium, P. aeruginosa*). In Gram-positive species the inventors observed an even lower conservation rate (Fig. 1c), with only 81 out of 725 unique interactions (11.2%) conserved in at least two species (Fig. 1d). 29 interactions were conserved in all three species (4%). The inventors reasoned that the lower interspecies conservation in the inventors' screen could be driven by the strain and species selection in the two screens, as for Gram-negative species two closely-related enterobacteria were tested, *E. coli* and *S. typhimurium,* and exhibited the highest overlap of interactions. On the contrary, the interaction conservation rate of either of these two species with *P. aeruginosa* is similar to cross-species conservation rates that the inventors detected for Gram-positive bacteria. Indeed, when the inventors compared the interaction conservation rate and genome sequence percentage identity (based on 40 universal single-copy marker genes), the two were significantly correlated (Methods, Fig.1e).

Synergies were shown to be more conserved in Gram-negative bacteria. Although the inventors observed a similar trend for Gram-positive species, this remained non-significant, even after removing non-antibiotic drugs for which intracellular targets and their conservation are unknown. Conserved synergies with Gram-positive species were mostly driven by drugs targeting the same essential and highly conserved cellular processes, such as DNA biosynthesis and translation (Fig. 1d). Some of these interactions, such as the synergy between macrolides and tetracyclines or between quinolones of different generations, have been observed before in Gram-negative species thus pointing towards conserved relationships between the targets of these compounds. Similarly, the broad antagonism between drugs targeting DNA and protein synthesis (Fig. 1d) is conserved in Gram-negative bacteria, and is due to the alleviation of protein-DNA imbalance after treatment with any of the two antibiotics alone. Overall, the inventors detected 52 synergies and 66 antagonisms shared across the Gram-positive/-negative divide.

**Numerous previously unknown drug synergies for *S. aureus***

The inventors built separate interaction networks and grouped drugs according to their class or cellular target (Fig. 2a-b). Although individual drug-drug interactions were only rarely conserved (Fig. 1c), interactions between drug classes or targeted processes tended to be more coherent in all three species. This functional concordance became even clearer when comparing drugs based on their composite of interactions with all other drugs. Interaction-based clustering better recapitulated drug functional classes (Methods) than clustering based on chemical structures (Methods), suggesting that drug interactions capture more information on drug mode of action than their chemical features.

Since *S. aureus* is the most relevant Gram-positive species in the current AMR scenario, the inventors systematically screened literature for reported drug interactions in this species. Out of 331 unique interactions detected across the two *S. aureus* strains in the inventors' study, the inventors found only 31 to have been previously reported (Fig. 2c). 55 further interactions have been reported in other bacterial species. Even when excluding those, the inventors' dataset revealed novel synergies for *S. aureus,* of which more than half (n = 97) conserved in both strains. This confirms the importance of systematic testing when probing combinations, and that the combinatorial space is a largely unexplored, but promising reservoir for antimicrobial strategies.

Known interactions include many conserved synergies between drugs with the same targets (Fig. Id), such as synergies between DNA-biosynthesis inhibitors (e.g. the well-described synergy between sulfamethoxazole and trimethoprim), between protein-synthesis inhibitors (e.g. azithromycin-spectinomycin, clarithromycin-doxycycline, clarithromycin-linezolid), and between cell-wall targeting antibiotics (Fig. 2a-b). Among these latter, the inventors confirmed the known synergy between cefepime and teicoplanin (Tang, H.-J. et al. Cephalosporin-Glycopeptide Combinations for Use against Clinical Methicillin-Resistant Staphylococcus aureus Isolates: Enhanced In vitro Antibacterial Activity. Front. Microbiol. 8, 884 (2017), Lai, C.-C., Chen, C.-C., Chuang, Y.-C. & Tang, H.-J. Combination of cephalosporins with vancomycin or teicoplanin enhances antibacterial effect of glycopeptides against heterogeneous vancomycin-intermediate Staphylococcus aureus (hVISA) and VISA. Sci. Rep. 7, 41758 (2017)), and the inventors validated it against several MRSA (Methicillin-Resistant *S. aureus*) clinical isolates, including a strain resistant to the last-resort antibiotic tigecycline (Fig. 2d). When the inventors infected larvae of the greater wax moth *Galleria mellonella* with this MRSA strain, the combination protected the animals from succumbing to the infection in contrast to single drug treatments (Fig. 2e), confirming that the synergies are also effective *in vivo.*

Synergies between cell-wall targeting drugs and translation inhibitors are cornerstones of anti-infective therapy against Gram-positive bacteria. The inventors could recapitulate some of these synergies, but not the traditionally used combinations of beta-lactams and aminoglycosides. This is in line with previously reported concerns on the general effectiveness of such type of combinations, whose interaction outcome seems to be strikingly strain-specific (Bartash, R. & Nori, P. Beta-lactam combination therapy for the treatment of Staphylococcus aureus and Enterococcus species bacteremia: A summary and appraisal of the evidence. Int. J. Infect. Dis. 63, 7-12 (2017); Ida, T. et al. Antagonism between aminoglycosides and beta-lactams in a methicillin-resistant Staphylococcus aureus isolate involves induction of an aminoglycoside-modifying enzyme. Antimicrob. Agents Chemother. 46, 1516-1521 (2002); Vakulenko, S. B. & Mobashery, S. Versatility of aminoglycosides and prospects for their future. Clin. Microbiol. Rev. 16, 430-450 (2003); Paul, M., Lador, A., Grozinsky-Glasberg, S. & Leibovici, L. Beta lactam antibiotic monotherapy versus beta lactam-aminoglycoside antibiotic combination therapy for sepsis. Cochrane Database Syst. Rev. CD003344 (2014)). Other cell-wall targeting drugs may hold some unexplored potential: for instance, fosfomycin strongly synergized with a diverse range of protein-synthesis inhibitors (lincosamides, oxazolidinones, streptomycin). In addition to these known interactions, fosfomycin synergized with almost all classes of translation inhibitors, including macrolides, tetracyclines and spectinomycin. Therefore, combinations of fosfomycin, whose clinical application against MRSA has been proposed in the last decade, represents an underexploited therapeutic resource against *S. aureus.*

Among the 303 previously unknown interactions the inventors detected, 19 out of 23 tested were also confirmed in extended $8 \times 8$ checkerboard benchmarking assays (9 of which in both *S. aureus* strains). Interestingly, adjuvants, like clavulanic acid, or antibiotics used in clinics only in fixed-concentration combinations (trimethoprim and sulphonamides), exhibited a number of synergies with other drugs, unveiling a so-far unexplored space for new combinations. The inventors sought to validate the efficacy of one of these combinations (teicoplanin-trimethoprim) and demonstrated it against several MRSA clinical isolates *in vitro* (Fig. 2d) and *in vivo* in a G. *mellonella* infection model (Fig. 2e).

**Fundamental differences for target-specific synergies between Gram-positive and Gram-negative species**

Drugs belonging to the same class or targeting the same cellular process exhibited mainly synergistic interactions in all three species (Fig. 2a-b). Indeed, this bias for synergies between drugs targeting the same process was significant (Fig. 3a), in agreement with previous data on Gram-negative bacteria and epistatic genetic interactions in different microbes. By chemically or genetically targeting different facets of the same cellular process, such combinations bypass the inbuilt redundancy and robustness of biological processes. However, the targeted cellular processes that were more prone to synergies were remarkably different when comparing Gram-positive and Gram-negative species. Synergies between protein-synthesis inhibitors were specifically prevalent in Gram-positive species, whereas Gram-negative species were dominated by synergies between cell-wall inhibitors (Fig. 3b). Since the drugs between the two screens largely overlapped, and their targets are conserved in bacteria, the inventors decided to further investigate the underlying reason for this clear difference.

Protein-synthesis inhibitors are mostly used against Gram-positive bacteria, as they often cannot cross the outer membrane (OM) of Gram-negative bacteria. The inventors reasoned that in Gram-positive species, with no such permeability bottleneck, these drugs could synergize at their target level - the ribosome, as shown before by genetically perturbing translation. By contrast, in Gram-negative bacteria the OM permeability bottleneck likely masks such synergistic interactions and enriches for antagonisms, which are common when based on a decrease in drug intracellular concentrations. The inventors confirmed this hypothesis by using the OM-defective *E. coli* mutant lptD4213, which is hyperpermeable to hydrophobic antibiotics and detergents. Many of the interactions between macrolides and different classes of protein synthesis inhibitors became synergistic in this E. coli strain-background (Fig. 3c), demonstrating that drug uptake bottlenecks are an important parameter for antibiotic interactions.

While fosfomycin- and bacitracin-based interactions are mostly conserved within Gram-positive species and across the Gram-positive/-negative divide, interactions within beta-lactams are radically different between Gram-positive and -negative species, with synergies being quite rare in the former. Beta-lactams have different affinities to the various penicillin-binding proteins (PBPs) present in bacteria. Interestingly, the number and type of PBPs are largely different across bacterial species, so the inventors hypothesized that this redundancy (number of PBP paralogues) was driving the observed difference. Indeed, the number of synergies in each strain tested correlated with the number of PBPs reported in each species (Fig. 3d). The higher the number of PBPs, the higher the probability that combining beta-lactams with different affinities to the various PBPs will lead to a synergistic bypassing of the redundancy. While further studies are needed, the inventors hypothesize that this target redundancy is driving the synergies between beta-lactam antibiotics, and that the difference the inventors observed here between Gram-positive and -negative species likely depends on the number of PBPs in the species tested.

Altogether, these results support the concept that drug interactions mirror key properties of cellular networks, such as their functional modularity and redundancy, and reflect fundamental differences in cellular architecture and physiology across the Gram-positive/-negative divide.

**An underestimated reservoir of interactions between non-antibiotics and antibiotics**

The inventors' drug interaction screen included eight non-antibiotic drugs, which exhibited a similar interaction frequency (11%) as antibiotics (13%) (Fig. 4a). This motivated us to expand the panel of non-antibiotic drugs tested, and to explore the range of synergies and antagonisms antibiotics exhibit with commonly used non-antibiotic medications in *S. aureus,* the leading Gram-positive pathogen for AMR-attributable deaths. The inventors selected 44 drugs to include pharmaceuticals that can be co-administered with antibiotics in *S. aureus* infections or non-antibiotics with previously reported antibacterial activity against *S. aureus.* Altogether, the inventors covered 19 therapeutic classes, X sub-groups of the ATC classification (second level), testing each drug in a range of three concentrations and against the panel of 62 drugs of the initial screen (2728 drug-drug interactions, $4 \times 4$ dose matrix) in *S. aureus* DSM 20231. Concentrations were selected to fall within therapeutic ranges, except for drugs with possible topical use, where higher concentrations were used. Interactions were scored and benchmarked as for the main screen (Methods).

The inventors confidently detected 197 interactions in the extended screen (Fig. 4b), an interaction frequency that was lower (7.8%) than the one of the initial screen or the set of eight non-antibiotic drugs included therein (Fig. 4a). Since all eight non-antibiotic drugs included in the main screen were selected because they had reported antibacterial activity, the inventors reasoned that this could account for the lower interaction rate here. Indeed, for those drugs that had antibacterial activity on their own, the interaction frequency was double (12% as compared to 5.9%) (Fig. 4c). For all non-antibiotics tested in this work (n = 52), the inventors detected 140 synergies and 105 antagonisms mainly with antibiotics (Fig. 4d). A small number of interactions (22 synergies and 23 antagonisms) were found between two non-antibiotics. Synergies offer a so-far unexploited potential for drug repurposing, whereas antagonisms expose risks of decreasing the efficacy of antimicrobial treatments.

The therapeutic classes that exhibited the highest number of interactions were anti-inflammatory drugs (n = 7, of which four NSAIDs) and hormone analogues (n = 6) (Fig. 4d), whereas in terms of antibiotics, protein-synthesis inhibitors dominated the interactions (Fig. 4d). Interestingly, selective estrogen-receptor modulators (SERMs), such as the two triphenylethylene compounds tamoxifene and clomifene, shared their synergies with cell-wall acting drugs and their antagonism with streptomycin. Hormone analogues in general (n = 6) engaged in several synergies (n = 11) and antagonisms (n = 17), suggesting an understudied impact that such commonly-used drugs, and potentially their natural counterparts, may have on the efficacy of antibacterial therapies. For the anti-inflammatory drugs, only four interactions with acetylsalicylic acid were previously known: its synergy with cefuroxime and its antagonisms with ciprofloxacin, oxacillin and azithromycin. The inventors validated the synergy between ibuprofen and gentamicin also against MRSA clinical isolates, including strains resistant to last-resort antibiotics for MRSA such as linezolid, *in vitro* and *in vivo* in a G. *mellonella* infection model (Fig. 4e-f).

**The antiaggregant ticagrelor has a broad impact on *S. aureus* physiology that accounts for its promiscuous interactions with antibiotics**

Ticagrelor, an ADP-receptor-inhibitor antiplatelet agent acting on the adenosine P2Y12 receptor, had the highest number of interactions (n = 27) among the 44 non-antibiotics tested (Fig. 4d). Ticagrelor has been shown to improve clinical outcomes in patients with pneumonia and sepsis caused by Gram-positive bacteria (Storey, R. F. et al. Lower mortality following pulmonary adverse events and sepsis with ticagrelor compared to clopidogrel in the PLATO study. Platelets 25, 517-525 (2014); Sexton, T. R. et al. Ticagrelor Reduces Thromboinflammatory Markers in Patients With Pneumonia. JACC Basic Transl Sci 3, 435-449 (2018)). This effect has been supported by different degrees of evidence that ticagrelor activates platelets upon systemic infection, protects them from *S. aureus* toxin-mediated damage, modulates their antibacterial properties, and exerts direct bactericidal activity on *S. aureus* at very high concentrations. However, ticagrelor mode of action on *S. aureus* and interactions with other drugs have remained largely uncharacterized.

To gain insights into the mode of action and interaction of ticagrelor, the inventors used two-dimensional thermal proteome profiling (2D-TPP) (Becher, I. et al. Thermal profiling reveals phenylalanine hydroxylase as an off-target of panobinostat. Nat. Chem. Biol. 12, 908-910 (2016); Mateus, A. et al. Thermal proteome profiling in bacteria: probing protein state in vivo. Mol. Syst. Biol. 14, e8242 (2018); Mateus, A. et al. Thermal proteome profiling for interrogating protein interactions. Mol. Syst. Biol. 16, e9232 (2020)) in both lysate and whole cell samples to investigate the direct and indirect effects of the drug, respectively (Methods). The inventors observed a destabilization of a number of ATP- and GTP-binding enzymes and transporters in both the whole cell and the lysate (Fig. 5a-b), and the induction of many purine biosynthesis enzymes (PurC, PurD, PurE, PurF, PurK, PurL, PurM, PurN, PurQ) in live cells (Fig 5a). This is in agreement with ticagrelor being an ADP-receptor-inhibitor antiplatelet agent. Furthermore, the MIC of ticagrelor increased upon supplementation with adenosine, inosine or their combination, suggesting that ticagrelor interferes with purine metabolism in *S. aureus.*

As mentioned above, the clinically observed effects of ticagrelor during *S. aureus* infection have not been linked so far to a direct effect of ticagrelor on *S. aureus* virulence. The inventors discovered a pervasive impact of ticagrelor on *S. aureus* virulence determinants and regulators, many of which were down-regulated, and others destabilized (Fig 5a). In particular, the inventors observed destabilization in lysate and downregulation in whole-cell samples of key clotting factors secreted by *S. aureus* (ClfA, ClfB), the coagulase Coa and the von Willebrand-factor binding protein (vWBP) NWMN_075764. These effects, evident at a clinically-relevant ticagrelor concentration offer an alternative explanation for the beneficial effect of antiaggregant therapy as an adjuvant in *S. aureus* systemic infection.

Ticagrelor exhibited a number of synergies and antagonisms with antibiotics in MSSA (Methicillin-sensitive *S. aureus;* Fig. 4d). Interestingly, it broadly sensitized MSSA and MRSA to both cationic peptides (nisin) and antibiotics (aminoglycosides, such as gentamicin; Fig. 5c). This potentiation effect of aminoglycosides occurred at low ticagrelor concentrations and was also evident at the killing level (Fig. 5d) and *in vivo,* during infection of G. *mellonella* (Fig. 5e). Since aminoglycosides need energy to cross the membrane in most bacteria, the inventors wondered whether ticagrelor acted at that level, for example by modulating the cell surface charge and increasing aminoglycoside uptake. Consistently with this hypothesis, two proteins involved in the lipoteichoic acid (LTA) D-alanylation, DltC and DltD, were destabilized in the TPP lysate data, and TagG, a subunit of the cell wall teichoic acid (WTA) translocase, was destabilized in the whole cell sample (Fig 5a-b). Disruption of teichoic acids, and specifically, inactivation of dltA, dltB and dltC have been shown to sensitize *S. aureus* to cationic compounds because of an increase in the net negative charge of *S. aureus* surface. The inventors observed a decrease in the MIC of the aminoglycoside gentamicin and the cationic antibiotic nisin in IPTG-inducible CRISPRi knockdown mutants of both the dltABCD operon and of tagG (Methods, Fig. 5f). Ticagrelor treatment also increased the binding of positively-charged cytochrome C to intact *S. aureus* cells (Fig. 5g). Thus, ticagrelor treatment impacts the thermal stability, and presumably the activity of proteins involved in WTA flipping and LTA D-alanylation, leading to an increase in the surface net negative charge of *S. aureus,* and thereby potentiating the uptake of cationic antibiotics, such as aminoglycosides and nisin.

**Claims**

1. An antibacterial pharmaceutical composition for the treatment of a Gram-positive bacterial infection, comprising a combination of i) at least one drug selected from the group of ADP-receptor-inhibitor antiplatelet agents and antifolate antibiotics used in the clinic only in fixed concentrations and derivatives and pharmaceutically acceptable salts thereof with ii) at least one antibiotic selected from the group of aminoglycosides, cationic peptides, glycopeptides, and derivatives and pharmaceutically acceptable salts thereof.

2. The antibacterial pharmaceutical composition for the treatment of a Gram-positive bacterial infection according to claim 1, wherein the ADP-receptor-inhibitors antiplatelet agent is selected from ticagrelor and derivatives and pharmaceutically acceptable salts thereof.

3. The antibacterial pharmaceutical composition for the treatment of a Gram-positive bacterial infection according to claim 1 or 2, wherein the antifolate antibiotic used in the clinic only in fixed concentration is selected from trimethoprim, and derivatives and pharmaceutically acceptable salts thereof.

4. The antibacterial pharmaceutical composition for the treatment of a Gram-positive bacterial infection according to any one of claims 1 to 3, wherein the glycopeptide is selected from teicoplanin and derivatives and pharmaceutically acceptable salts thereof.

5. The antibacterial pharmaceutical composition for the treatment of a Gram-positive bacterial infection according to any one of claims 1 to 4, wherein the aminoglycoside is selected from gentamicin and derivatives and pharmaceutically acceptable salts thereof.

6. An antibacterial pharmaceutical composition for the treatment of a Gram-positive bacterial infection, comprising a combination of teicoplanin and trimethoprim and derivatives and pharmaceutically acceptable salts thereof.

7. An antibacterial pharmaceutical composition for the treatment of a Gram-positive bacterial infection, comprising a combination of ticagrelor and gentamicin and derivatives and pharmaceutically acceptable salts thereof.

8. The antibacterial pharmaceutical composition for the treatment of a Gram-positive bacterial infection according to any one of claims 1 to 7, wherein the bacterial infection is by *S. aureus,* and antibiotic-resistant strains thereof, such as MRSA.

9. The pharmaceutical composition according to any one of claims 1 to 8 for use in the prevention and/or treatment of a bacterial infection, wherein said bacterial infection is selected from an infection of the gastrointestinal tract, intra-abdominal infection, colitis, an infection of the urogenital tract, an infection of the upper and lower respiratory tract, rhinitis, tonsillitis, pharyngitis, bronchitis, pneumonia, an infection of the inner organs, nephritis, pyelonephritis, peritonitis, endocarditis, an infection of the central nervous system, meningitis, osteomyelitis, an infection of the eyes, an infection of the ears, acute bacterial otitis media, a cutaneous infection, a subcutaneous infection, a burn wound infection, a wound infection, a skin disorder, toxic shock syndrome, bacteremia, sepsis, pelvic inflammatory disease, an intravascular infection, a bone infection, a joint infection, a deep-seated abscess, and a medical implant infection.

10. The pharmaceutical composition for use according to claim 9, wherein said bacterial infection is caused by a Gram-positive bacterium, preferably wherein said Gram-positive bacterial infection is by *S. aureus,* and antibiotic-resistant strains thereof, such as MRSA.

11. The pharmaceutical composition for use according to claim 9 or 10, wherein said combination of said pharmaceutical composition is administered to a subject simultaneously, separately, or sequentially, wherein said subject is a mammal, such as a human, preferably a human patient, optionally wherein said composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, nasal agent, inhalation mixture, aerosol, mouthwash, mouth spray, nose spray.

12. The pharmaceutical composition for use according to any one of claims 9 to 11, wherein said use furthermore prevents the development and/or spread of bacterial antibiotic resistance.

13. A method for producing an antibacterial pharmaceutical composition according to any one of claims 1 to 8, comprising the step of formulating said combination as selected into an antibacterial pharmaceutical composition for the treatment of a Gram-positive bacterial infection.

Figure 1

Figure 1 (continued)

Figure 1 (continued)

**d** Conserved interactions in Gram-positive bacteria

d = 47
l = 81

protein synthesis
DNA
oxidative stress/PMF
membrane
non-antibiotic
cell wall

#drug-drug interactions/ class-class pair
1    15

#drugs/class
3   5   10   15   17

**e**

$R = 0.97, p = 1.2e-09$

% marker–gene–based identity

Fraction of conserved interactions

Species pair
Gram–negative/Gram–negative
Gram–positive/Gram–negative
Gram–positive/Gram–positive

Figure 2

Figure 2 (continued)

Figure 2 (continued)

**d**

MRSA, Tig-R

**e**

MRSA, Tig-R

Figure 3

Figure 3 (continued)

c

Figure 3 (continued)

d

Figure 4

**Main Screen**
(*S. aureus* Newman, DSM 20231,
*B. subtilis, S. pneumoniae*)

**Non-antibiotic Screen**
(*S. aureus* DSM 20231)

Figure 4 (continued)

**Non-antibiotic Screen**
(*S. aureus* DSM 20231)

c

**MRSA, Lzd-R**

e

Figure 4 (continued)

Figure 4 (continued)

Figure 5

Figure 5 (continued)

**c**                    **MRSA, Tig-R**

**d**                    **MRSA USA300**

Figure 5 (continued)

Figure 5 (continued)

**g**

Europäisches Patentamt
European Patent Office
Office européen des brevets

# EUROPEAN SEARCH REPORT

Application Number

EP 22 20 7154

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TRAUB WALTER H. ET AL: "Teicoplanin Combined with Various Antibiotics and Human Blood against a Multiple-Drug-Resistant Strain of Staphylococcus aureus", CHEMOTHERAPY, vol. 37, no. 3, 1 January 1991 (1991-01-01), pages 186-195, XP093035646, CH ISSN: 0009-3157, DOI: 10.1159/000238852 Retrieved from the Internet: URL:http://dx.doi.org/10.1159/000238852> | 1-6,8-13 | INV.<br>A61K38/14<br>A61K31/505<br>A61K31/519<br>A61K31/7036<br>A61K45/06<br>A61P31/04 |
| Y | * abstract * | 2 | |
| X | ZINNER S. H. ET AL: "Synergism of trimethoprim combined with aminoglycosides in vitro and in serum of volunteers", EUROPEAN JOURNAL OF CLINICAL MICROBIOLOGY, vol. 1, no. 3, 1 June 1982 (1982-06-01), pages 144-148, XP093035676, ISSN: 0722-2211, DOI: 10.1007/BF02019614 Retrieved from the Internet: URL:http://link.springer.com/article/10.10 07/BF02019614/fulltext.html> * abstract * | 1,3-6, 8-13 | |

TECHNICAL FIELDS SEARCHED (IPC)

A61K
A61P

-/--

The present search report has been drawn up for all claims

| Place of search | Date of completion of the search | Examiner |
|---|---|---|
| Munich | 30 March 2023 | Pilling, Stephen |

EPO FORM 1503 03.82 (P04C01)

Europäisches
Patentamt

European
Patent Office

Office européen
des brevets

## EUROPEAN SEARCH REPORT

**Application Number**

EP 22 20 7154

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PAISLEY J. W. ET AL: "Synergistic Activity of Gentamicin with Trimethoprim or Sulfamethoxazole-Trimethoprim Against Escherichia coli and Klebsiella pneumoniae", ANTIMICROBIAL AGENTS AND CHEMOTHERAPY, vol. 14, no. 5, 1 November 1978 (1978-11-01), pages 656-658, XP093035680, US ISSN: 0066-4804, DOI: 10.1128/AAC.14.5.656 Retrieved from the Internet: URL:https://journals.asm.org/doi/pdf/10.1128/AAC.14.5.656> * abstract * * Discussion * | 1,3-6, 8-13 | |
| X | CN 102 784 158 A (SICHUAN DERUNTONG BIOLOG TECHNOLOGY CO LTD) 21 November 2012 (2012-11-21) * abstract * | 1,3-6, 8-13 | |
| X | US 4 888 327 A (EDWARDS JOHN G [US]) 19 December 1989 (1989-12-19) * claim 1; example 1 * | 1,3-6, 8-13 | **TECHNICAL FIELDS SEARCHED (IPC)** |

-/--

The present search report has been drawn up for all claims

| Place of search | Date of completion of the search | Examiner |
|---|---|---|
| Munich | 30 March 2023 | Pilling, Stephen |

EPO FORM 1503 03.82 (P04C01)

| | Europäisches Patentamt European Patent Office Office européen des brevets | **EUROPEAN SEARCH REPORT** | Application Number EP 22 20 7154 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOMBERT M E ET AL: "Synergism of imipenem and amikacin in combination with other antibiotics against Nocardia asteroides", ANTIMICROBIAL AGENTS AND CHEMOTHERAPY, vol. 24, no. 5, 1 November 1983 (1983-11-01), pages 810-811, XP093035725, US ISSN: 0066-4804, DOI: 10.1128/AAC.24.5.810 Retrieved from the Internet: URL:https://journals.asm.org/doi/pdf/10.11 28/AAC.24.5.810> * column 1 second paragraph and the final paragraph; table 2 * | 1,3-6, 8-13 | |
| X | BENES JIRI ET AL: "Listerial Endocarditis in a Penicillin-allergic Woman Successfully Treated with a Combination of 4 Drugs", SCANDINAVIAN JOURNAL OF INFECTIOUS DISEASES., vol. 34, no. 5, 8 January 2002 (2002-01-08), pages 383-384, XP093035657, NO ISSN: 0036-5548, DOI: 10.1080/00365540110080430 Retrieved from the Internet: URL:http://dx.doi.org/10.1080/003655401100 80430> * abstract * | 1,3-6, 8-13 | TECHNICAL FIELDS SEARCHED (IPC) |

-/--

The present search report has been drawn up for all claims

| Place of search | Date of completion of the search | Examiner |
|---|---|---|
| Munich | 30 March 2023 | Pilling, Stephen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 22 20 7154

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LANCELLOTTI PATRIZIO ET AL: "Antibacterial Activity of Ticagrelor in Conventional Antiplatelet Dosages Against Antibiotic-Resistant Gram-Positive Bacteria", JAMA CARDIOLOGY, vol. 4, no. 6, 1 June 2019 (2019-06-01), page 596, XP093035894, ISSN: 2380-6583, DOI: 10.1001/jamacardio.2019.1189 | 1-6,8-13 | |
| Y | * abstract * | 1-6,8,13 | |
| X | RAMOS-MARTÍN V ET AL: "Pharmacodynamics of teicoplanin against MRSA", JOURNAL OF ANTIMICROBIAL CHEMOTHERAPY, vol. 72, no. 12, 1 December 2017 (2017-12-01), pages 3382-3389, XP093035900, GB ISSN: 0305-7453, DOI: 10.1093/jac/dkx289 Retrieved from the Internet: URL:https://academic.oup.com/jac/article-pdf/72/12/3382/21898969/dkx289.pdf> | 1-6,8-13 | |
| Y | * abstract * | 1-6,8-13 | |

-/--

TECHNICAL FIELDS
SEARCHED (IPC)

The present search report has been drawn up for all claims

| Place of search | Date of completion of the search | Examiner |
|---|---|---|
| Munich | 30 March 2023 | Pilling, Stephen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**EUROPEAN SEARCH REPORT**

| | Application Number |
|---|---|
| | EP 22 20 7154 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GRIM SHELLEE A. ET AL: "Trimethoprim-Sulfamethoxazole as a Viable Treatment Option for Infections Caused by Methicillin-Resistant Staphylococcus aureus", PHARMACOTHERAPY, vol. 25, no. 2, 5 January 2005 (2005-01-05), pages 253-264, XP093035907, US ISSN: 0277-0008, DOI: 10.1592/phco.25.2.253.56956 Retrieved from the Internet: URL:http://dx.doi.org/10.1592/phco.25.2.253.56956> | 1-6,8-13 | |
| Y | * abstract * | 1-6,8-13 | |
| X | COSGROVE SARA E. ET AL: "Initial Low-Dose Gentamicin for Staphylococcus aureus Bacteremia and Endocarditis Is Nephrotoxic", CLINICAL INFECTIOUS DISEASES, vol. 48, no. 6, 15 March 2009 (2009-03-15), pages 713-721, XP093035917, US ISSN: 1058-4838, DOI: 10.1086/597031 Retrieved from the Internet: URL:https://academic.oup.com/cid/article-pdf/48/6/713/799852/48-6-713.pdf> | 1-6,8-13 | |
| Y | * abstract * | 1-6,8-13 | **TECHNICAL FIELDS SEARCHED (IPC)** |

The present search report has been drawn up for all claims

| Place of search | Date of completion of the search | Examiner |
|---|---|---|
| Munich | 30 March 2023 | Pilling, Stephen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 7154

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2023

| Patent document cited in search report | | Publication date | Patent family member(s) | Publication date |
|---|---|---|---|---|
| CN 102784158 | A | 21-11-2012 | NONE | |
| US 4888327 | A | 19-12-1989 | NONE | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019206781 A

**Non-patent literature cited in the description**

- **TYERS, M. ; WRIGHT, G. D.** Drug combinations: a strategy to extend the life of antibiotics in the 21st century. *Nat. Rev. Microbiol.,* 2019, vol. 17, 141-155
- **TRAUB WH et al.** Teicoplanin Combined with Various Antibiotics and Human Blood against a Multiple-Drug-Resistant Strain of Staphylococcus aureus. *Chemotherapy.,* 1991, vol. 37 (3), 186-95
- **WU W et al.** Teicoplanin combined with conventional vancomycin therapy for the treatment of pulmonary methicillin-resistant Staphylococcus aureus and Staphylococcus epidermidis infections. *World J Clin Cases.,* 06 December 2021, vol. 9 (34), 10549-10556
- *Antimicrob Agents Chemother.,* July 2005, vol. 49 (7), 2735-45
- **LANCELLOTTI P et al.** Antibacterial Activity of Ticagrelor in Conventional Antiplatelet Dosages Against Antibiotic-Resistant Gram-Positive Bacteria. *JAMA Cardiol.,* 01 June 2019, vol. 4 (6), 596-599
- *Antimicrobial Resistance Collaborators. Lancet.,* 12 February 2022, vol. 399 (10325), 629-655
- **BLISS, C. I.** THE TOXICITY OF POISONS APPLIED JOINTLY. *Ann. Appl. Biol.,* 1939, vol. 26, 585-822
- **EJIM, L. et al.** Combinations of antibiotics and non-antibiotic drugs enhance antimicrobial efficacy. *Nat. Chem. Biol.,* 2011, vol. 7, 348-350
- **BROCHADO, A. R. et al.** Species-specific activity of antibacterial drug combinations. *Nature,* 2018, vol. 559, 259-263
- **SUN, J. et al.** Repurposed drugs block toxin-driven platelet clearance by the hepatic Ashwell-Morell receptor to clear Staphylococcus aureus bacteremia. *Sci. Transl. Med.,* 2021, vol. 13
- **PHANCHANA, M. et al.** Repurposing a platelet aggregation inhibitor ticagrelor as an antimicrobial against Clostridioides difficile. *Sci. Rep.,* 2020, vol. 10, 6497
- **STOREY, R. F. et al.** Lower mortality following pulmonary adverse events and sepsis with ticagrelor compared to clopidogrel in the PLATO study. *Platelets,* 2014, vol. 25, 517-525

- **LANCELLOTTI, P. et al.** Antibacterial Activity of Ticagrelor in Conventional Antiplatelet Dosages Against Antibiotic-Resistant Gram-Positive Bacteria. *JAMA Cardiol,* 2019, vol. 4, 596-599
- **MAIER, L. et al.** Unravelling the collateral damage of antibiotics on gut bacteria. *Nature,* 2021
- **MURRAY, C. J. L. et al.** Global burden of bacterial antimicrobial resistance in 2019: a systematic analysis. *Lancet,* 2022, vol. 399, 629-655
- **KAVCIC, B. ; TKACIK, G. ; BOLLENBACH, T.** Mechanisms of drug interactions between translation-inhibiting antibiotics. *Nat. Commun.,* 2020, vol. 11, 4013
- **MARTIN B ; GARCIA P ; CASTANIÉ M-P ; CLAVERYS J-P.** The recA gene of Streptococcus pneumoniae is part of a competence-induced operon and controls lysogenic induction. *Molecular Microbiology,* 1995, vol. 15, 367-379
- **HUBER, P. J.** Robust Estimation of a Location Parameter. *Ann. Math. Stat.,* 1964, vol. 35, 73-101
- **BLISS, C. I.** THE TOXICITY OF POISONS APPLIED JOINTLY. *Ann. Appl. Biol.,* 1939, vol. 26, 585-615
- **GOLDONI, M. ; JOHANSSON, C.** A mathematical approach to study combined effects of toxicants in vitro: evaluation of the Bliss independence criterion and the Loewe additivity model. *Toxicol. In Vitro,* 2007, vol. 21, 759-769
- **BAJUSZ, D. ; RACZ, A. ; HÉBERGER, K.** Why is Tanimoto index an appropriate choice for fingerprint-based similarity calculations?. *J. Cheminform.,* 2015, vol. 7, 20
- **MORGAN, H. L.** The Generation of a Unique Machine Description for Chemical Structures-A Technique Developed at Chemical Abstracts Service. *J. Chem. Doc.,* 1965, vol. 5, 107-113
- **ROGNES, T. ; FLOURI, T. ; NICHOLS, B. ; QUINCE, C. ; MAHE, F.** VSEARCH: a versatile open-source tool for metagenomics. *PeerJ,* 2016, vol. 4, e2584
- **MATEUS, A. et al.** Thermal proteome profiling in bacteria: probing protein state in vivo. *Mol. Syst. Biol.,* 2018, vol. 14, e8242

- **BECHER, I. et al.** Thermal profiling reveals phenylalanine hydroxylase as an off-target of panobinostat. *Nat. Chem. Biol.,* 2016, vol. 12, 908-910
- **KURZAWA, N. et al.** *Computational analysis of ligand dose range thermal proteome profiles.,* 2020
- **TENENBAUM D ; M. B. KEGGREST.** *Client-side REST access to the Kyoto Encyclopedia of Genes and Genomes (KEGG),* 2021
- **GEISTLINGER, L. ; CSABA, G. ; ZIMMER, R.** Bioconductor's EnrichmentBrowser: seamless navigation through combined results of set- & network-based enrichment analysis. *BMC Bioinformatics,* 2016, vol. 17, 45
- **WU, T. et al.** clusterProfiler 4.0: A universal enrichment tool for interpreting omics data. *Innovation (N Y),* 2021, vol. 2, 100141
- **RADLINSKI, L. C. et al.** Chemical Induction of Aminoglycoside Uptake Overcomes Antibiotic Tolerance and Resistance in Staphylococcus aureus. *Cell Chem Biol,* 2019, vol. 26, 1355-1364.e4
- **JAWETZ, E.** The use of combinations of antimicrobial drugs. *Annu. Rev. Pharmacol.,* 1968, vol. 8, 151-170
- **TANG, H.-J. et al.** Cephalosporin-Glycopeptide Combinations for Use against Clinical Methicillin-Resistant Staphylococcus aureus Isolates: Enhanced In vitro Antibacterial Activity. *Front. Microbiol.,* 2017, vol. 8, 884
- **LAI, C.-C. ; CHEN, C.-C. ; CHUANG, Y.-C. ; TANG, H.-J.** Combination of cephalosporins with vancomycin or teicoplanin enhances antibacterial effect of glycopeptides against heterogeneous vancomycin-intermediate Staphylococcus aureus (hVISA) and VISA. *Sci. Rep.,* 2017, vol. 7, 41758
- **BARTASH, R. ; NORI, P.** Beta-lactam combination therapy for the treatment of Staphylococcus aureus and Enterococcus species bacteremia: A summary and appraisal of the evidence. *Int. J. Infect. Dis.,* 2017, vol. 63, 7-12
- **IDA, T. et al.** Antagonism between aminoglycosides and beta-lactams in a methicillin-resistant Staphylococcus aureus isolate involves induction of an aminoglycoside-modifying enzyme. *Antimicrob. Agents Chemother.,* 2002, vol. 46, 1516-1521
- **VAKULENKO, S. B. ; MOBASHERY, S.** Versatility of aminoglycosides and prospects for their future. *Clin. Microbiol. Rev.,* 2003, vol. 16, 430-450
- **PAUL, M. ; LADOR, A. ; GROZINSKY-GLASBERG, S. ; LEIBOVICI, L.** Beta lactam antibiotic monotherapy versus beta lactam-aminoglycoside antibiotic combination therapy for sepsis. *Cochrane Database Syst. Rev.,* 2014
- **SEXTON, T. R. et al.** Ticagrelor Reduces Thromboinflammatory Markers in Patients With Pneumonia. *JACC Basic Transl Sci,* 2018, vol. 3, 435-449
- **MATEUS, A. et al.** Thermal proteome profiling for interrogating protein interactions. *Mol. Syst. Biol.,* 2020, vol. 16, e9232